(19)

Europäisches
Patentamt
European
Patent Office
Office européen
des brevets

(11) **EP 3 266 725 A1**

(12) **EUROPEAN PATENT APPLICATION**
published in accordance with Art. 153(4) EPC

(43) Date of publication:
**10.01.2018 Bulletin 2018/02**

(21) Application number: **16758641.1**

(22) Date of filing: **02.03.2016**

(51) Int Cl.:
**B65D 47/18** (2006.01)   **A61J 1/05** (2006.01)
**B65D 47/06** (2006.01)

(86) International application number:
**PCT/JP2016/001135**

(87) International publication number:
**WO 2016/139943 (09.09.2016 Gazette 2016/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **02.03.2015   JP 2015040619
19.03.2015   JP 2015056833
08.05.2015   JP 2015095401
08.05.2015   JP 2015095402**

(71) Applicant: **Toyo Seikan Group Holdings, Ltd.
Shinagawa-ku
Tokyo 141-8627 (JP)**

(72) Inventors:
• **NAGAO, Jotaro**
  **Yokohama-shi**
  **Kanagawa 240-0062 (JP)**

• **OKAMOTO, Kota**
  **Yokohama-shi**
  **Kanagawa 240-0062 (JP)**
• **IWASAKI, Tsutomu**
  **Yokohama-shi**
  **Kanagawa 240-0062 (JP)**
• **AOYA, Masaki**
  **Yokohama-shi**
  **Kanagawa 240-0062 (JP)**
• **KIMURA, Satoo**
  **Yokohama-shi**
  **Kanagawa 240-0062 (JP)**

(74) Representative: **Liebetanz, Michael
Isler & Pedrazzini AG
Giesshübelstrasse 45
Postfach 1772
8027 Zürich (CH)**

(54) **NOZZLE**

(57)   A reduction in dripping quantity of a container for instillation of eye drops is realized, and deterioration or the like of dripping performance is not caused by repeated use, whereby stable small-quantity dripping can be conducted reliably, and the front end part of the nozzle can be protected by a cap without fail. Provided is a nozzle 10 of a container 1 for instillation of eye drops that is formed of a plastic molded body composed of a non-fluorine-based resin in which fluorine atoms are incorporated into a molecular chain of a non-fluorine-based resin constituting a plastic molded body and a front end part 11 of the nozzle 10 is roughened.

Fig.1

## Description

Technical Field

The present invention relates to a nozzle provided in a pouring part of a container for instillation of eye drops, for example, which is capable of dripping a liquid inside a container little by little.

Background Art

Generally, in a container for instillation of eye drops, etc., a nozzle is provided in the pouring part so that the liquid (eye drops) in the container can be dripped little by little.

Here, usually, the human eye has a volume to hold about 20 $\mu$l of a tear fluid, but with a nozzle of a conventional container for instillation of eye drops, the dripping quantity of one droplet is generally about 30 to 40 $\mu$l, and there was a problem that almost half of the dripped eye drops are overflown from the eye.

Therefore, proposals have been made on a nozzle of a container for instillation of eye drops that enables dripping of eye drops in a smaller quantity corresponding to the tear-retaining volume of such human eyes.

For example, in Patent Document 1, proposed is "an instillation container" in which the front end of the dispensing nozzle for dripping eye drops from the container is provided with a needle part having an outer diameter of 0.5 mm or more and 2.5 mm or less such that the dripping quantity of one drop can be about 5 to 25 $\mu$l.

Further, Patent Document 2 proposes a "container for a liquid having a water-repellent nozzle" in which a liquid-repellent substance is applied to a nozzle tip sealed part inside the cap of the chemical solution container and when the container is closed, the liquid-repellent substance is transferred to the nozzle side, whereby the front end of the nozzle has liquid repellency, thereby enabling control the dripping quantity of one drop.

Related Art Documents

Patent Documents

Patent Document 1: WO2014/123140
Patent Document 2: JP-A-2011-105339

Summary of the Present invention

Problems to be Solved by the Present invention

In the "instillation container" described in Patent Document 1, a small needle part is provided at the front end of the nozzle so as to reduce the quantity of dripping. However, there is no liquid-repellent performance on the needle part itself, and droplets adhere to and remain on the nozzle front end part including a needle part while dripping is performed, and stable quantitative dripping cannot be performed as it is used repeatedly.

Further, a fine needle of 0.5 to 2.5 mm has a possibility of giving a user who uses an eye drop a fear that the needle pierces the eye since the front end looks very pointy.

Also, even with such a fine needle, the dripping quantity is limited to about 10 $\mu$l at most and, hence it cannot be said that it is sufficient in respect of small-quantity dripping.

On the other hand, in the "liquid container" described in Patent Document 2, a liquid-repelling substance is applied to the front end of the nozzle via a cap, and a substance different from the eye drop is applied to the front end of the nozzle of the eye drop container, problems such as contamination due to the entering of foreign matters into the container and adverse effects on the human body are easily concerned. Further, as in the case of Patent Document 1, there is a problem that stable quantitative dripping cannot be conducted after repeated use.

Therefore, this container was basically problematic as a container for instillation of eye drops, and it was thought that practical implementation was impossible, and hence, it could not be the means for solving the problems associated with the above-mentioned conventional containers for instillation of eye drops.

The present invention has been made in order to solve the above-mentioned problems associated with the conventional technology, and is aimed at providing a nozzle that realizes reduction in dripping quantity in a container for instillation of eye drops, can prevent liquid dripping or presence of residual liquid on the nozzle top surface, is free from contamination of a nozzle front end, mixing in of foreign matters or microorganism due to returning of a liquid from the nozzle to the container main body, and problems such as deterioration of dripping performance, etc. do not occur, and hence stable small-quantity dripping can be conducted stably without causing variations in dripping quantity, and further, the nozzle front end can be protected without fail.

Means for Solving the Problem

In order to attain the above-mentioned object, the nozzle of the present invention is a nozzle composed of a non-fluorine-based resin, wherein fluorine atoms are incorporated into a molecular chain of the non-fluorine-based resin constituting a surface of the nozzle.

Further, the nozzle of the present invention is a nozzle wherein a surface of a front end part of the nozzle is provided with a first surface positioned nearer to the center of the nozzle and a second surface continuing to the outer peripheral side of the first surface, and the first surface and the second surface are composed of surfaces differing in surface free energy.

Advantageous Effects of the Present invention

According to the present invention, reduction in dripping quantity in a container for instillation of eye drops is realized, liquid dripping or presence of a residual liquid on the nozzle top surface can be prevented, contamination of the nozzle front end, mixing in of foreign matters or microorganisms due to returning of a liquid from the nozzle to the container main body, deterioration in dripping performance due to repeated use, etc. can be eliminated. Also, occurrence of problems such as deterioration in dripping performance by repeated use can be prevented.

In addition, no variations in dripping quantity are caused, and small-quantity dripping can be conducted stably without fail, and further, the nozzle front end part can be protected by a cap without fail.

Due to such advantageous effects, a nozzle that is preferable as a container for instillation of eye drops can be realized, for example.

Brief Description of the Drawings

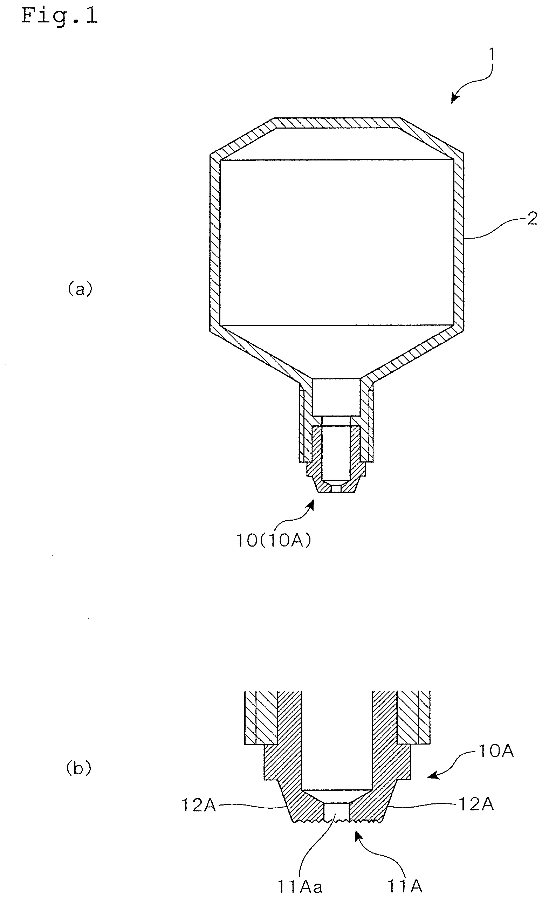

Fig. 1 shows a nozzle according to a first embodiment of the present invention, in which (a) is a cross-sectional view of an entire container for instillation of eye drops, and (b) is an enlarged cross-sectional view of a front end part of the nozzle shown in (a);

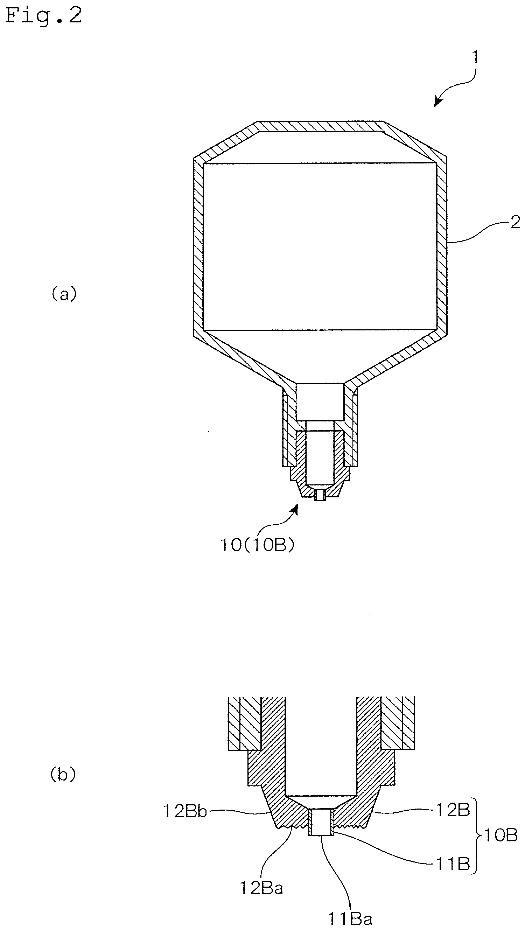

Fig. 2 shows a nozzle according to a second embodiment of the present invention, in which (a) is a cross-sectional view of the entire container for instillation of eye drops, and (b) is an enlarged cross-sectional view of a front end part of the nozzle shown in (a);

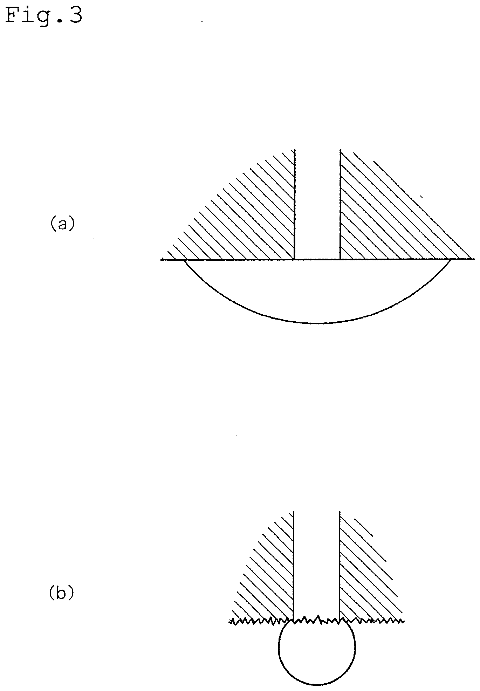

Fig. 3 is an explanatory view schematically showing a state of liquid droplets of a nozzle according to the presence or absence of liquid-repellent treatment, in which (a) shows a case of a nozzle which is not subjected to a liquid-repellent treatment, and (b) shows a case of a nozzle which is subjected to a liquid-repellent treatment;

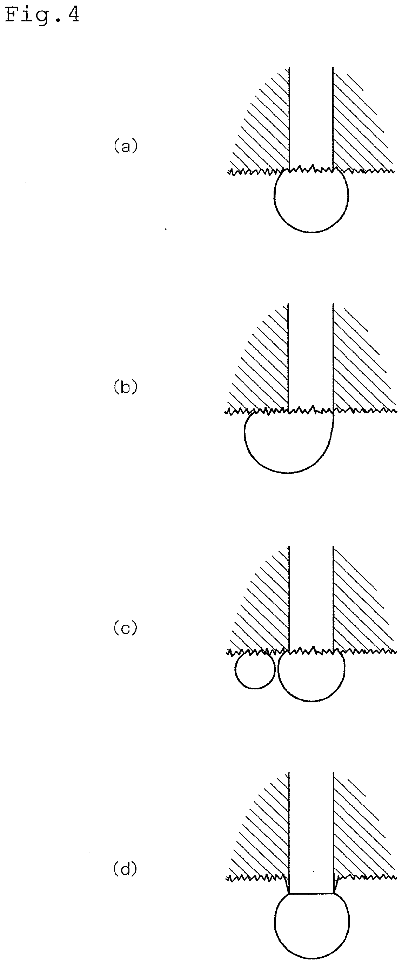

Fig. 4 is an explanatory diagram schematically showing a variation in the quantity of dripping in a nozzle subjected to a liquid-repellent treatment, in which (a) shows a normal case, (b) shows a case where liquid repellency is biased, (c) shows a case where air is entrained in liquid droplets, (d) shows a case where circumferential protruded parts (burr) are present on an opening of a nozzle subjected to a liquid-repellent treatment;

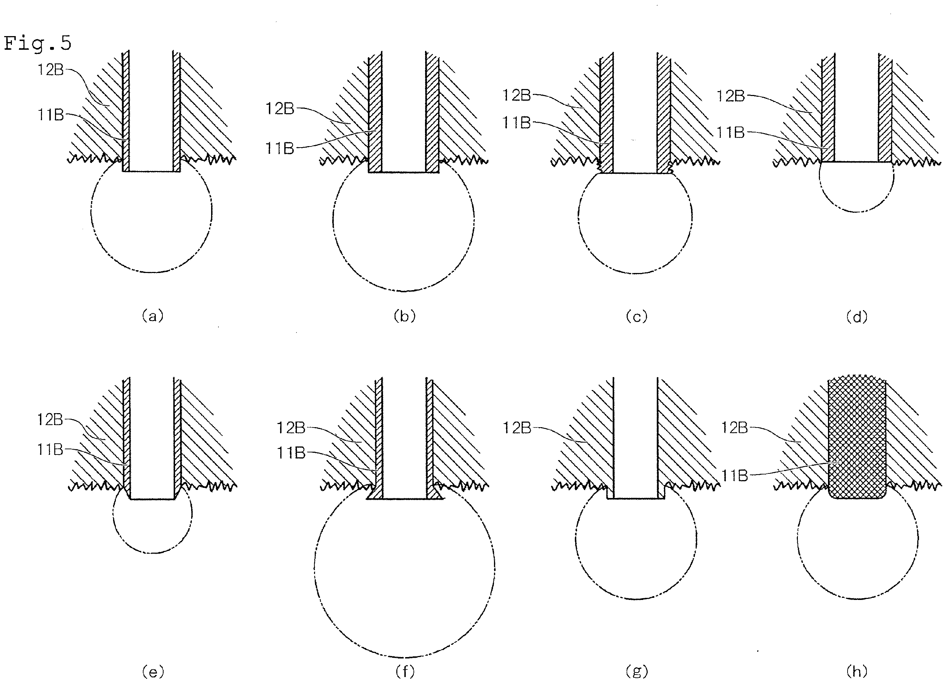

Fig. 5 is an explanatory view schematically showing an embodiment of a first/second surfaces of a nozzle front end part according to a second embodiment of the present invention, in which (a) shows a case in which a cylindrical first dripping part is allowed to protrude from the front end of a second dripping part positioned on its outer periphery to constitute the first/second surfaces; (b) shows a case where the area of the first surface is enlarged by allowing the cylindrical part constituting the first dripping part shown in (a) to have an increased thickness; (c) shows a case where the side surface of the first dripping part shown in (b) is subjected to a liquid-repellent treatment as in the case of the second surface; (d) shows a case where the first/second surfaces are constituted without protruding the first dripping part from the front end of the second dripping part; (e) shows a case where the front end of the first dripping part shown in (a) is tapered and the first surface is inclined towards the dripping direction; (f) shows a case where the front end of the first dripping part shown in (a) is formed in a trumpet form, thereby to enlarge the area of the first surface and to allow it to be protruded to the second surface side; (g) shows a case where the first/second surfaces are integrally formed on the front end side of the second dripping part; and (h) shows a case where a first dripping part is constituted by using a fiber member, an unwoven fabric, etc. instead of the first cylindrical dripping part shown in (a);

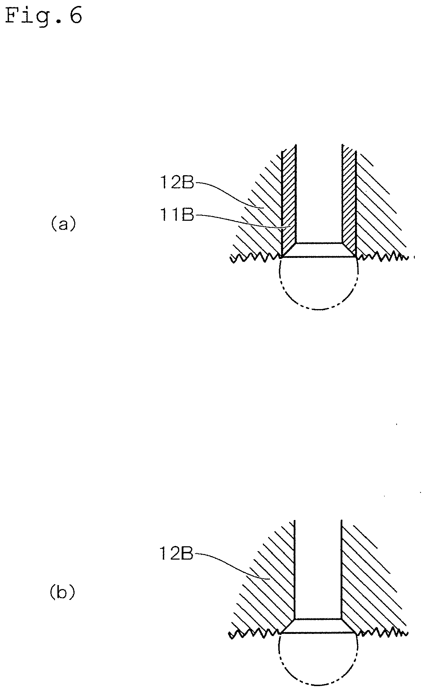

Continuing from Fig. 5, Fig. 6 is an explanatory view schematically showing an embodiment of the first/second surfaces of the nozzle front part according to the second embodiment of the present invention, in which (a) shows a case where the first dripping part in which the inner surface of the front end part is chamfered is arranged without being protruded from the front end of the second dripping part, thereby allowing the part with a chamfered shape to be a first surface; and (b) shows a case where the first surface in a chamfered shape shown in (a) is integrally formed with the second surface on the front end part of the second dripping part;

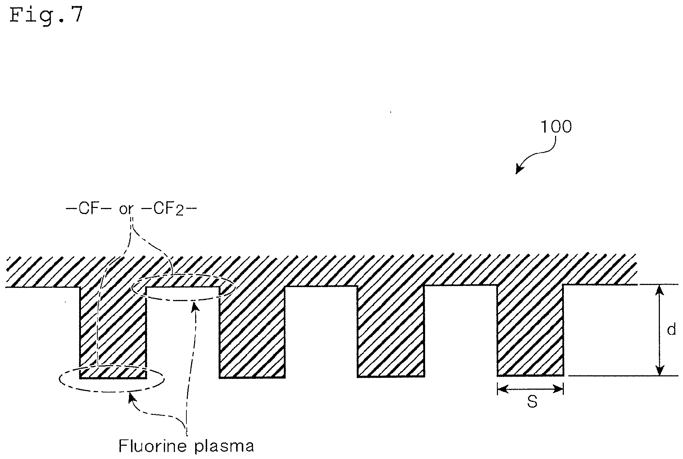

Fig. 7 is an explanatory view schematically showing the morphology of the liquid-repellent roughened surface formed on the front end part of the nozzle according to the present invention;

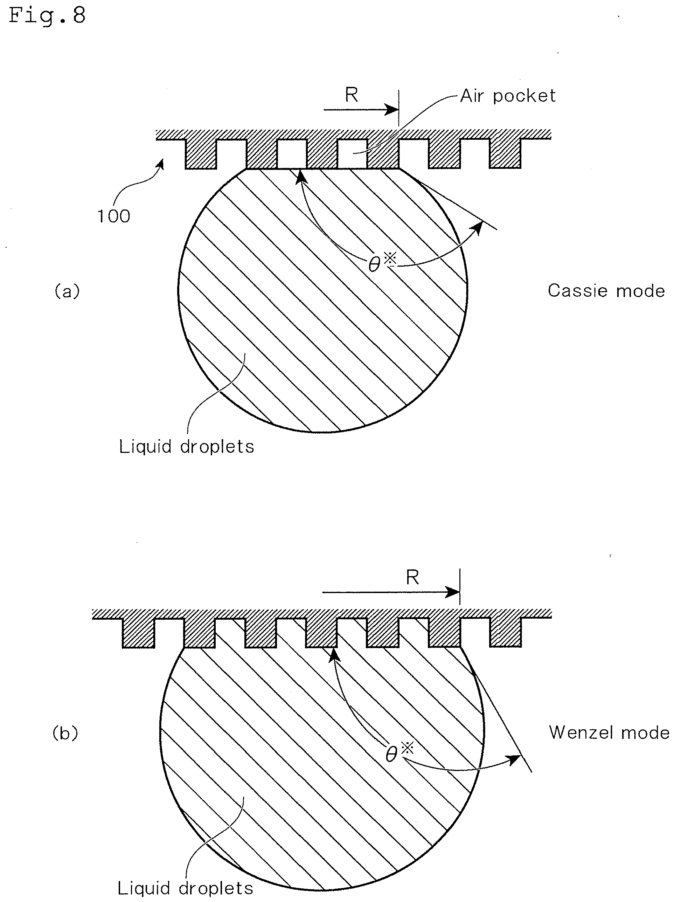

Fig. 8 is an explanatory view schematically showing the contact pattern of liquid droplets on the roughened surface shown in Fig. 7 in the Cassie-Baxter model and the Wenzel model;

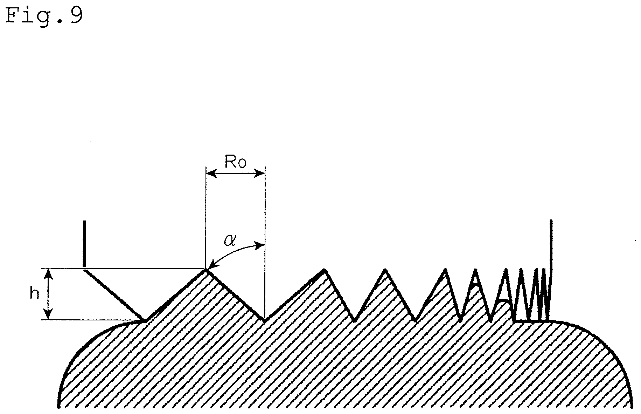

Fig. 9 is an explanatory enlarged view showing another morphology of a roughened surface formed on the front end part of the nozzle according to the present invention;

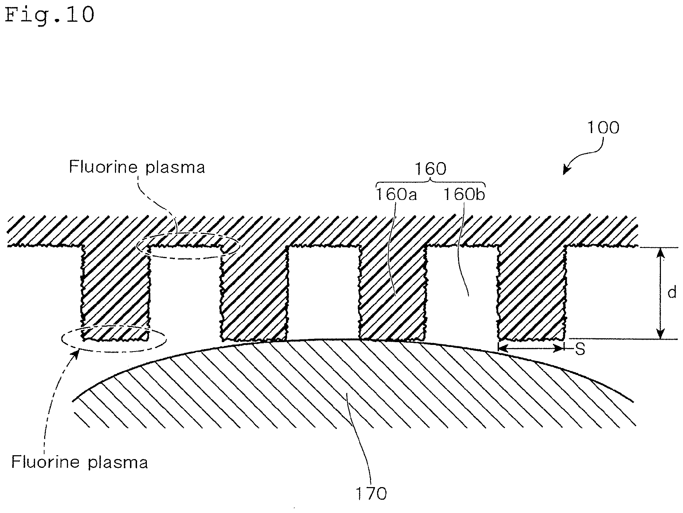

Fig. 10 is an explanatory enlarged view showing the morphology of a preferable roughened surface formed on the front end part of the nozzle according to the present invention;

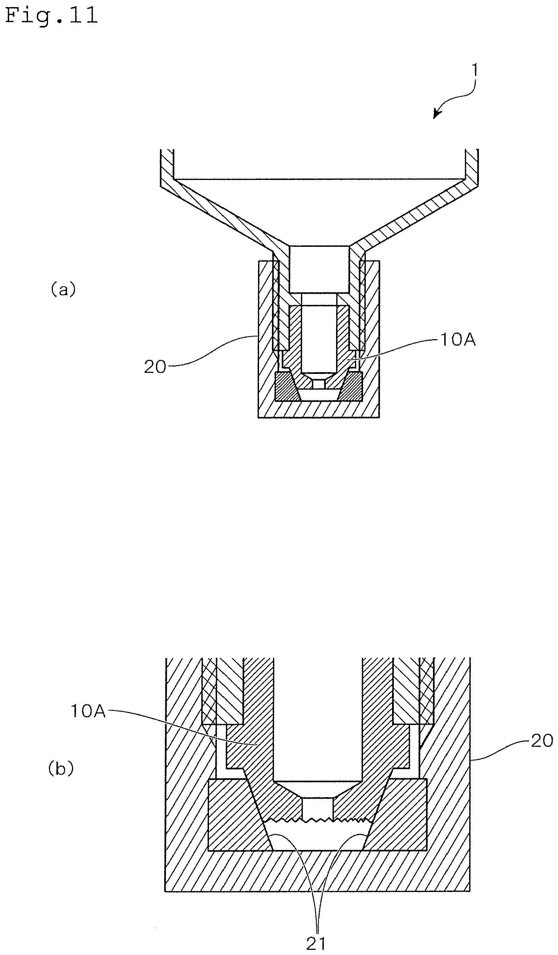

Fig. 11 is a view showing a cap covering the nozzle according to the first embodiment of the present invention, in which (a) is a cross-sectional view of essential part of the container for instillation of eye drops with the cap being attached; and (b) is an enlarged cross-sectional view of the front end part of the nozzle of the cap shown in (a);

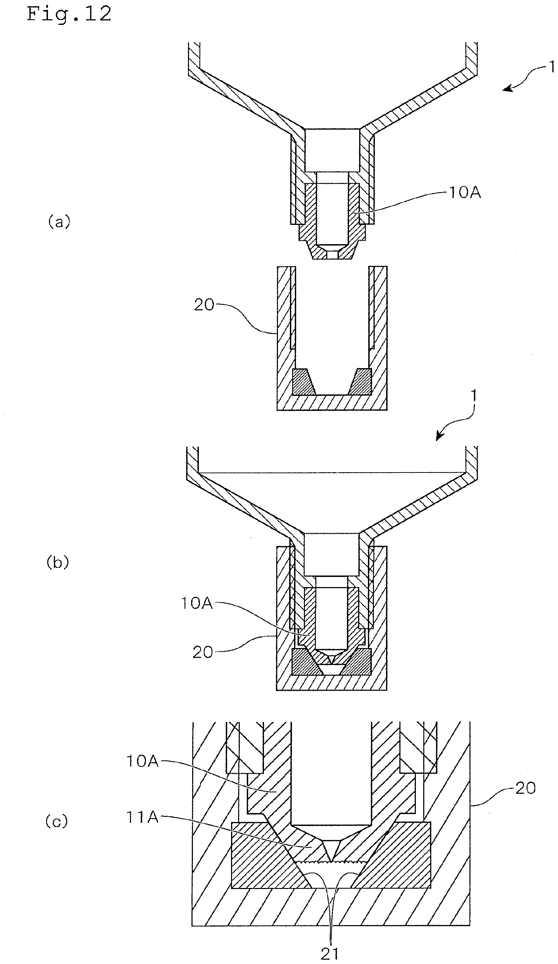

Fig. 12 is a view showing another morphology of a cap according to the first embodiment of the present invention, in which (a) is a cross-sectional view of an essential part of the container for instillation of eye drops with the cap being detached; and (b) is an enlarged cross-sectional view of the container for instillation of eye drops with the cap being attached; and (c) is an enlarged cross-sectional view of the front end part of the nozzle of the cap shown in (b);

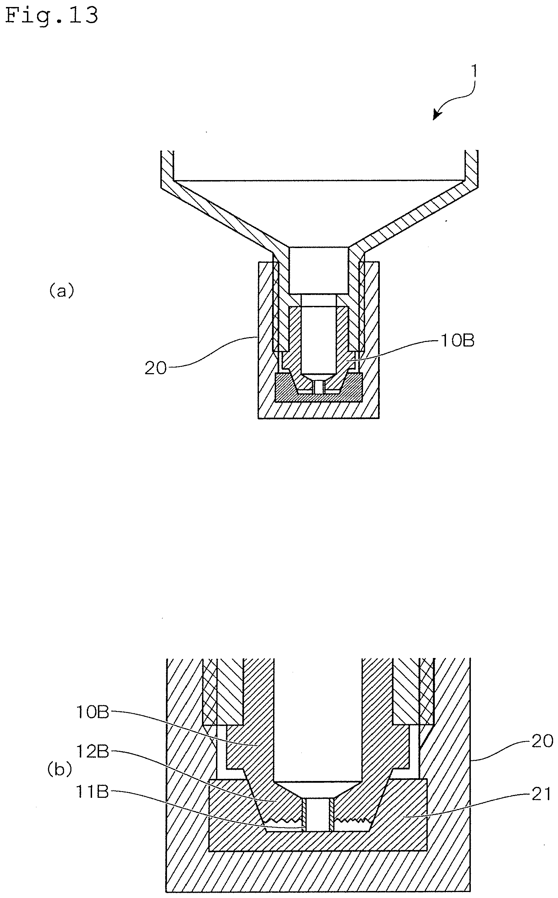

Fig. 13 shows a cap covering the nozzle according to the second embodiment of the present invention, in which (a) is a cross-sectional view of an essential part of the container for instillation of eye drops with the cap being attached; and (b) is an enlarged cross-sectional view of the front end part of the nozzle of the cap shown in (a);

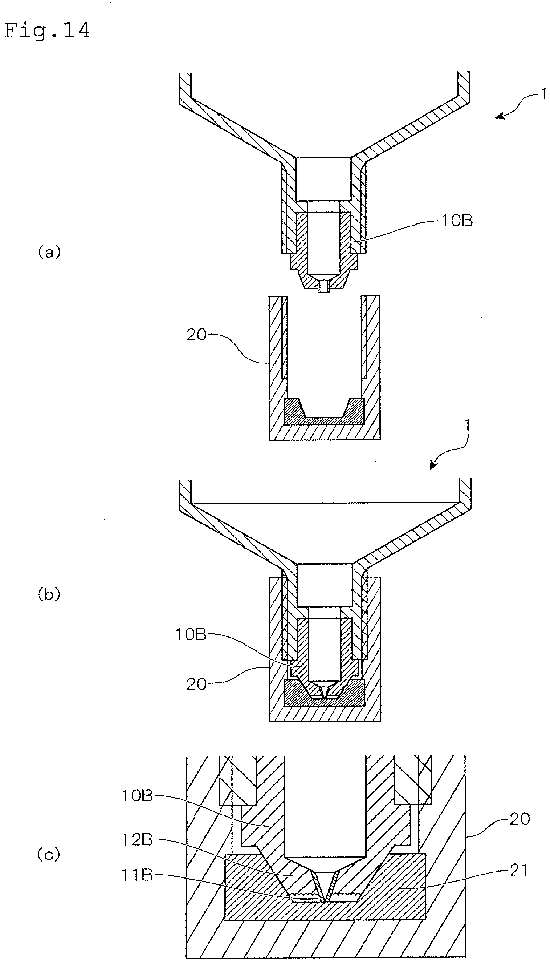

Fig. 14 shows another morphology of the cap covering the nozzle according to the second embodiment of the present invention, in which (a) is a cross-sectional view of an essential part of the container for instillation of eye drops with the cap being detached; and (b) is an enlarged cross-sectional view of the container for instillation of eye drops with the cap being attached; and (c) is a cross-sectional view of the front end part of the nozzle of the cap shown in (b);

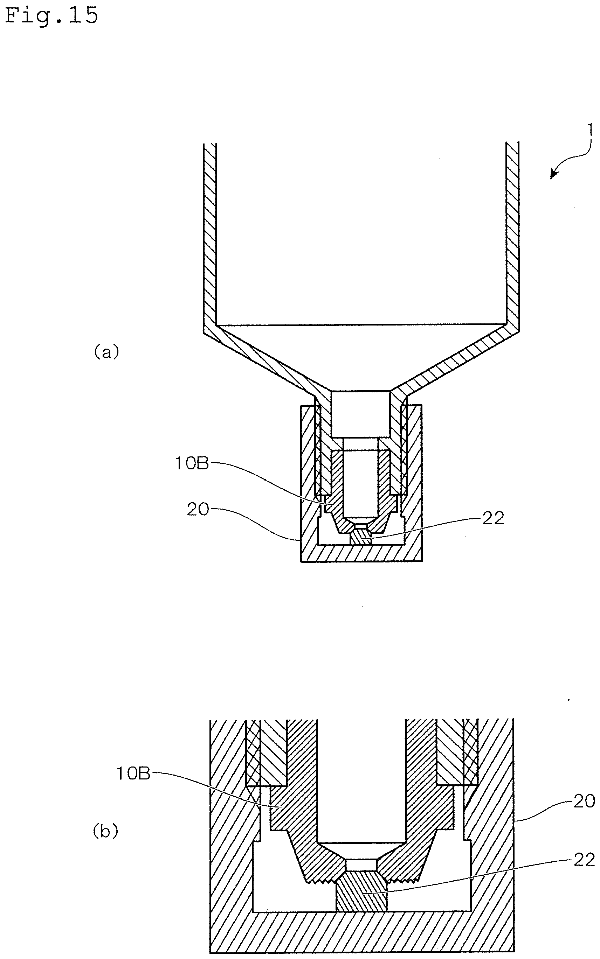

Fig. 15 is still another morphology of the cap covering the nozzle according to the second embodiment of the present invention, in which (a) is a cross-sectional view of an essential part of the container for instillation of eye drops with the cap being attached; and (b) is an enlarged cross-sectional view of the front end part of the nozzle of the cap shown in (a);

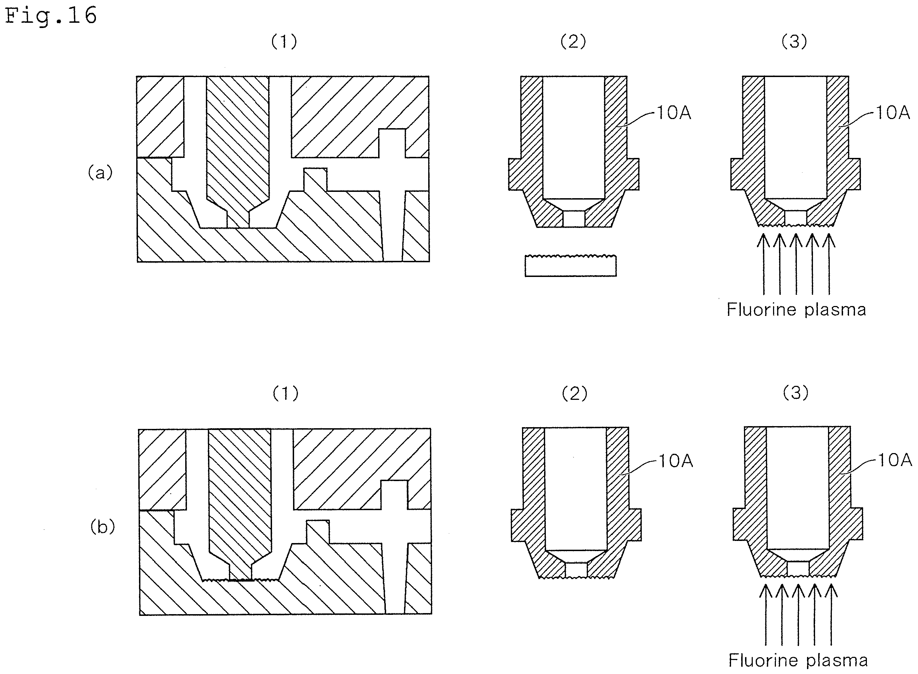

Fig. 16 is an explanatory view schematically showing the method for producing a nozzle according to the first embodiment of the present invention, in which (a) is a case where common injection molding is used, and (b) is a case where injection compression molding or heat & cool type injection molding is used;

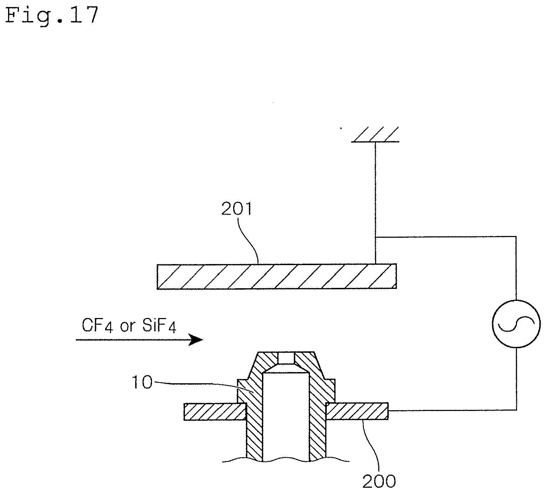

Fig. 17 is an explanatory view schematically showing the method for fluorine plasma etching treatment for roughening the front end part of the nozzle according to the present invention;

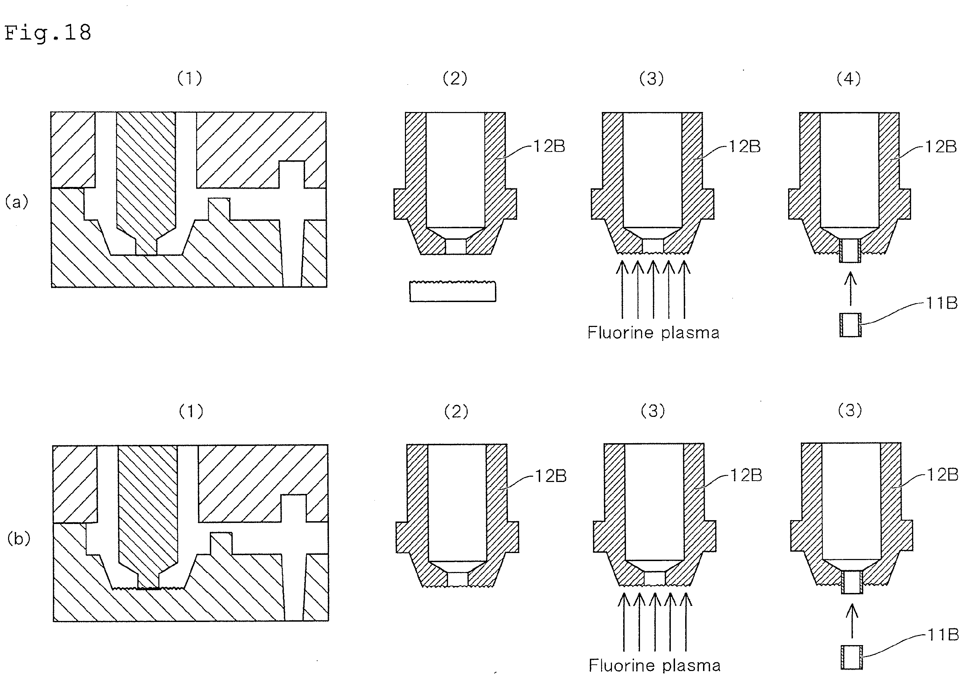

Fig. 18 is an explanatory view schematically showing the method for producing the nozzle according to the second embodiment of the present invention, in which (a) is a case where common injection molding is used; and (b) is a case where injection compression molding or heat & cool type injection molding is used;

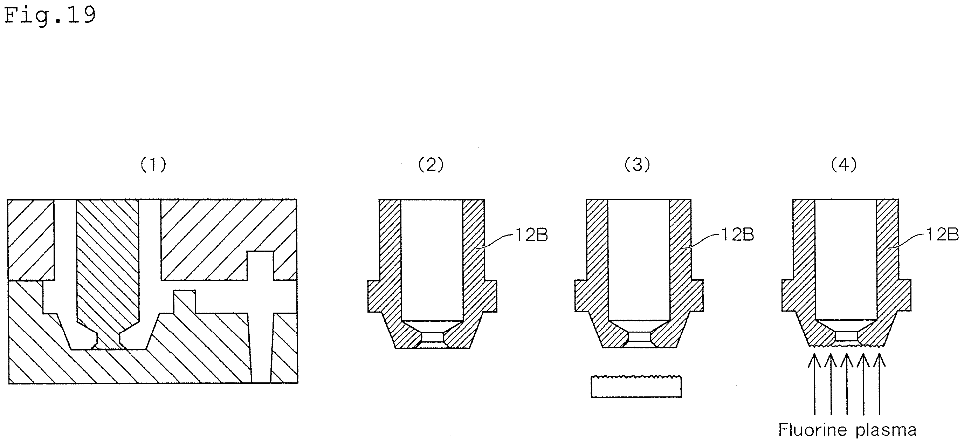

Fig. 19 is an explanatory view schematically showing the method for producing the nozzle according to the second embodiment of the present invention, and showing a case where, in the production method shown in Fig. 18(a), a first surface with a chamfered shape is formed on the front end opening of the second dripping part without using the first dripping part;

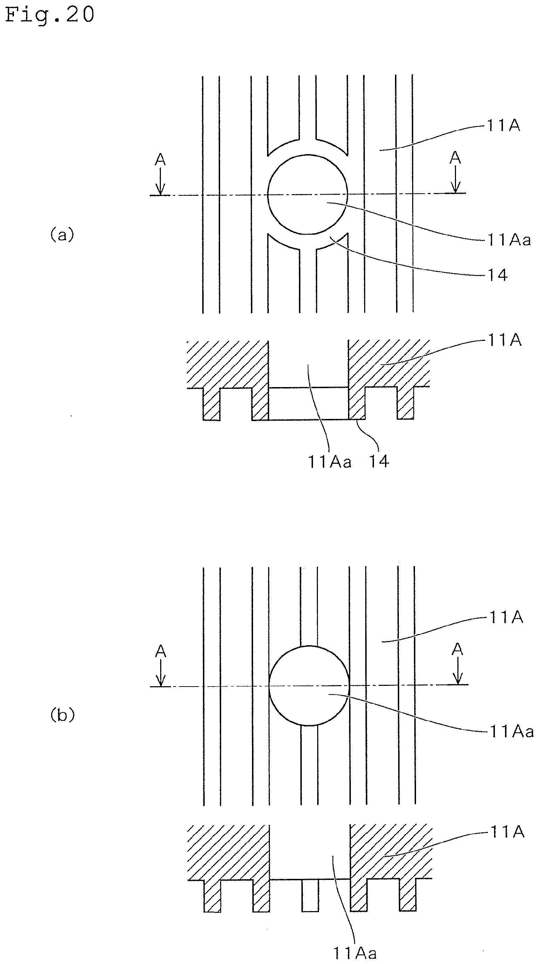

Fig. 20 is a plan view and a cross-sectional view taken along the line A-A thereof of the front end part when a bulwark is provided on the periphery of the opening of the nozzle according to the first embodiment of the present invention, in which (a) shows a case where a bulwark is provided on the periphery of the opening, and (b) shows a case where a bulwark is not provided;

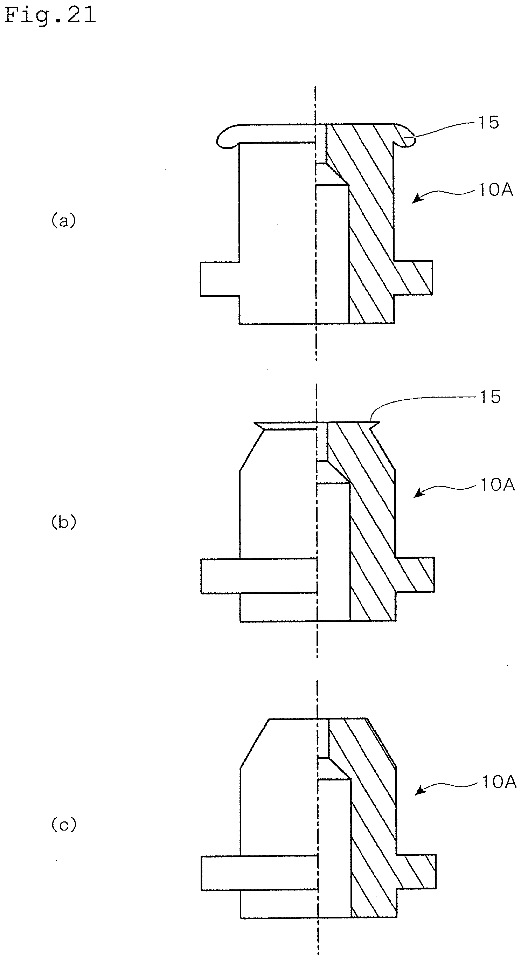

Fig. 21 is a partial cross-sectional view of the nozzle in which a thick wall part is provided on the outer periphery of the front end part of the nozzle according to the first embodiment of the present invention, in which (a) shows a case where the thick wall part is overhung relative to the top surface of the nozzle front end part; (b) shows a case where the thick wall part is slanted relative to the top surface of the nozzle front end part; and (c) shows a nozzle in which no thick wall part is provided;

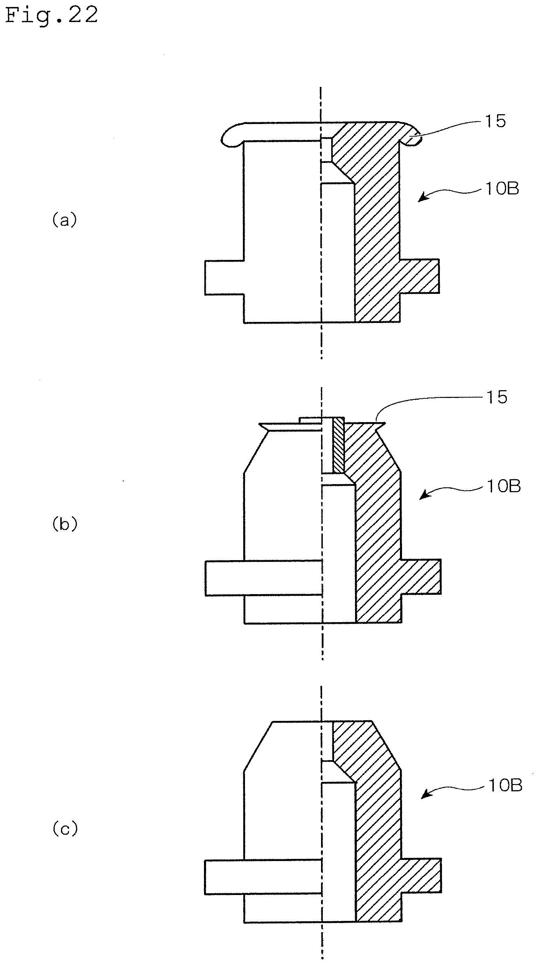

Fig. 22 is a partial cross-sectional view of the nozzle in which a thick wall part is provided on the outer periphery of the front end part of the nozzle according to the second embodiment of the present invention, in which (a) shows a case where the thick wall part is overhung relative to the top surface of the nozzle front end part; (b) shows a case where the thick wall part is slanted relative to the top surface of the nozzle front end part; and (c) shows a nozzle in which no thick wall part is provided;

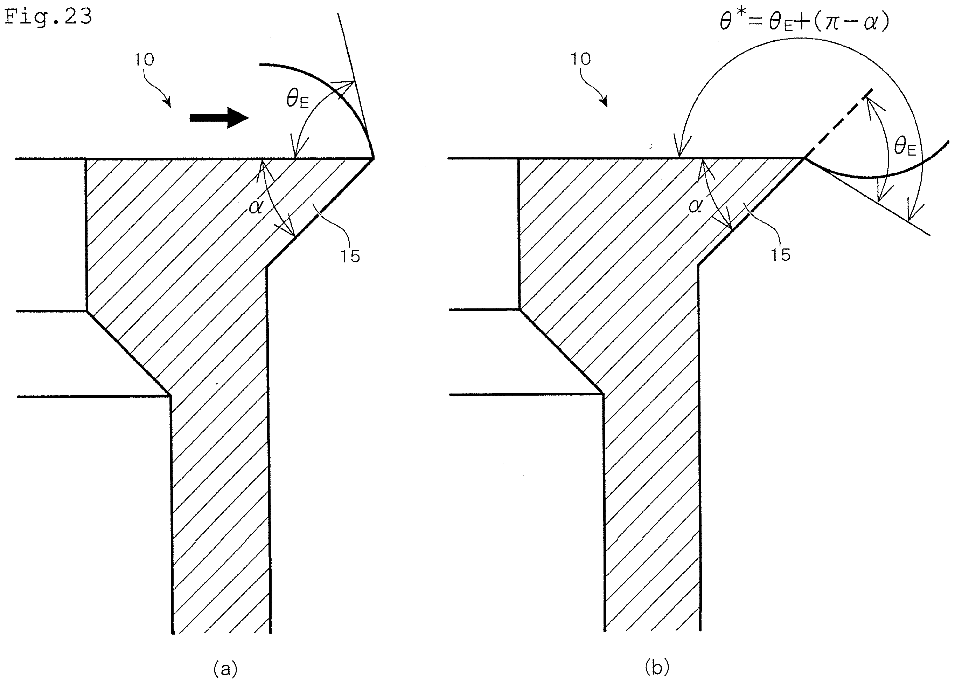

Fig. 23 is a cross-sectional view of an essential part of the nozzle for explaining the drainability when a slanted thick wall part is provided on the outer periphery of the nozzle front end part;

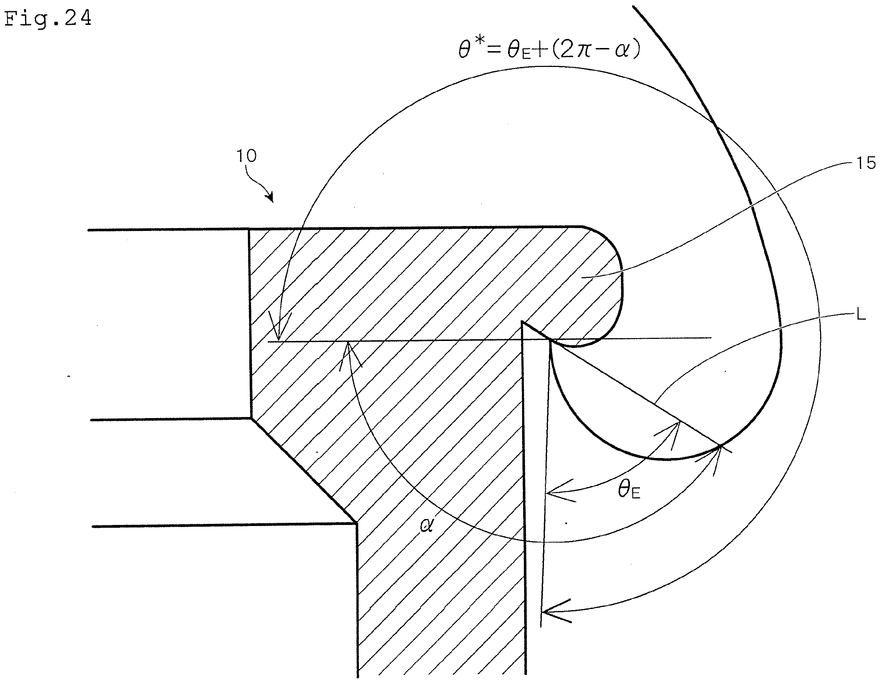

Fig. 24 is a cross-sectional view of an essential part of the nozzle for explaining the drainability when an overhung thick wall part is provided on the outer periphery of the nozzle front end part; and

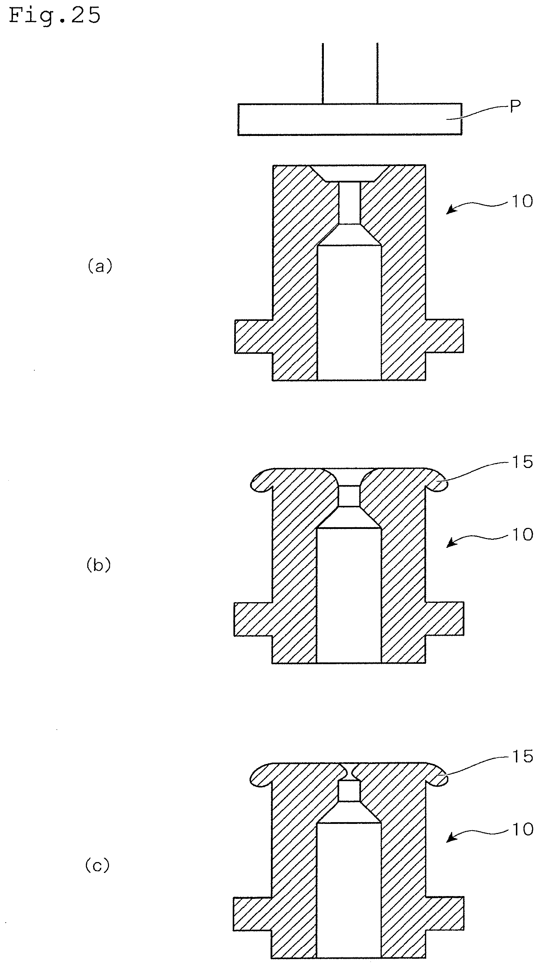

Fig. 25 is an explanatory view schematically showing the method for producing a thick wall part by heat pressing in the nozzle according to the present invention, in which (a) shows a state prior to heat pressing in which the opening (discharge port) of the nozzle is made large in advance such that it is not blocked by heat pressing; (b) shows a state after heat pressing; and (c) shows a state in which the opening of the nozzle is blocked by heat pressing.

Mode for Carrying out the Present invention

Herein below, the embodiment of the nozzle of the present invention will be explained with reference to the drawings.

Fig. 1 shows a nozzle according to a first embodiment of the present invention, in which (a) is a cross-sectional view of an entire container for instillation of eye drops, and (b) is an enlarged cross-sectional view of a front end part of the nozzle shown in (a).

Similarly, Fig. 2 shows a nozzle according to a second embodiment of the present invention, in which (a) is a cross-sectional view of the entire container for instillation of eye drops, and (b) is an enlarged cross-sectional view of a front end part of the nozzle shown in (a).

[Container for instillation for eye drops]

As shown in Fig. 1, the nozzle according to the embodiment of the present invention constitutes a nozzle 10 that serves as a pouring port of a container 1 for instillation of eye drops.

Specifically, the container 1 for instillation of eye drops includes a container main body 2 capable of accommodating and storing a liquid serving as an eye drop in the inside thereof, and nozzles 10 (10A, 10B) that protrude from almost the center of the upper surface (bottom surface when an eye drop is dripped) of this container main body 2. The container main body 2 and the nozzle 10 are intercommunicated, whereby the eye drops stored in the container main body 2 can be poured and dripped from the opening at the front end part of the nozzle 10.

[Nozzle]

As shown in Fig. 1 and Fig. 2, the nozzles 10 (10A, 10B) are formed separately from the container main body 2, and inserted into and engaged with a protruded part for attaching a nozzle formed in the container main body 2 to be integrated with the container main body 2 to form the container 1 for instillation of eye drops.

Specifically, the nozzle 10 is formed in a cylindrical shape, for example, and is intercommunicated with the storing space of the container main body 2. Then, through the opening of the front end part of the cylindrical nozzle 10, a liquid is poured and dropped from the inside of the container main body 2.

Further, in the container main body 2 including the nozzle 10, a cap 20 mentioned later is detachably attached (see Figs. 11 to 15). By provision of such cap 20, the nozzle 10 is covered and the inside of the container main body 2 is sealed and the front end part of the nozzle 10 is protected.

Specifically, on the surface of the protruded part of the container main body 2 on which the nozzle 10 is attached, a screw structure that screws with the inner surface of the cap 20 is provided, whereby the cap 20 is detachably attached by screwing to the container main body 2, and the container main body 2 is sealed in a state that the cap 20 is attached.

As mentioned later, in the nozzle 10A according to the first embodiment, as shown in Fig. 1, the side surface part 12A continuing the front end part 11A of the nozzle 10A is formed in a tapered shape that inclines towards the front end part 11A, and the container main body 2 is sealed when this side surface 12A contacts and is pressed by the liner 21 of the cap 20.

On the other hand, in the nozzle 10B according to the second embodiment, as shown in Fig. 2, the side surface part 12Bb of the second dripping part 12B that constitutes the front end part of the nozzle 10B is formed in a tapered shape that inclines towards the front end. The side surface part 12Bb of the second dripping part 12B and the front end part of the first dripping part 11B (first surface 11Ba) contacts with and is pressed by a liner 21 on the inside of the cap 20, whereby the entire container main body 2 is sealed.

For the details of the cap 20, an explanation will be made later with reference to Figs. 11 to 15.

The container main body 2 and the nozzle 10, including a cap 20 mentioned later, are formed of a prescribed plastic material.

The plastic material forming the container main body 2 and the nozzle 10 is not particularly restricted, and can be formed of various thermoplastic resins (e.g. olefin-based resin such as polyethylene and polypropylene) or a polyester resin represented by polyethylene terephthalate (PET).

In particular, as for the nozzle 10 according to the present invention, as mentioned later, on the surface of the nozzle front end part (front end part 11A, second surface 12Ba), a roughened surface 100 composed of concavo-convex surface is formed (see Figs. 7 to 10). Therefore, in respect of shape stability, strength, etc. of the roughened surface 100, it is preferred that a polyolefin-based resin such as polyethylene and polypropylene which is a non-fluorine-based resin) be used.

By using such plastic resin material, the container main body 2 and the nozzle 10 can be formed by using a known technology such as injection molding.

Since the container main body 2 and the nozzle 10 are formed as separate bodies (separate parts), the container main body 2 can also be formed of a non-plastic material such as glass or metal. In addition, the container main body 2 and the nozzle 10 can be integrally formed by integral molding such as a blow-fill seal molding method.

The method for molding the nozzle 10 of the present embodiment will be explained later with reference to Figs. 16, 18 to 20.


[Liquid-repellency treatment]

In the nozzle 10 according to the embodiment of the present invention, by applying a predetermined liquid-repellency treatment to the nozzle front end part, the liquid contained in the container body 2 can be reliably and stably poured and dripped from the nozzle front end part in a desired quantity of droplets.

Fig. 3 is an explanatory view schematically showing a state of liquid droplets of a nozzle according to the presence or absence of liquid-repellent treatment, in which (a) shows a case where a nozzle is not subjected to a liquid-repellent treatment, and (b) shows a case where a nozzle is subjected to a liquid-repellent treatment;

First, as shown in Fig. 3(a), if the surface of the front end part of the nozzle is not subjected to a liquid-repellency treatment yet (e.g. the surface of a bulk plastic resin as it is), since the liquid repellency of the surface is "low", droplets poured from the nozzle are adhered to the surface of the nozzle front end part and are spread almost hemispherically. The liquid spreading to the front end of the nozzle does not separate from the nozzle surface unless the amount becomes considerable. As a result, if more droplets than desired are dripped and spread to such a degree that the inner diameter of the nozzle can be ignored, it becomes difficult to control the quantity of liquid droplets depending on the inner diameter of the nozzle.

On the other hand, as shown in Fig. 3(b), in the case where the surface of the front end part of the nozzle is subjected to a liquid-repellent treatment, if the surface of a plastic resin is subjected to a fluorinating treatment or a surface roughing treatment, since the liquid repellency of the surface is "high", the droplets poured from the nozzle become almost spherical without wetting and spreading the front end of the nozzle. Then, when the weight of the droplets becomes higher than the adhesiveness between the liquid droplets and the nozzle front end part, the liquid droplets are removed from the nozzle surface and fall down and are dripped. Since the droplets do not spread while wetting, the adhesiveness is low, and only a small quantity of droplets are dripped. Further, by setting the inner diameter of the nozzle to a prescribed dimension, it is possible to pour and drip a prescribed quantity of liquid droplets.

However, even when the nozzle surface is fluorinated or roughened to increase the liquid repellency, there may be a case that there are variations in the dripping quantity of the poured droplets.

Fig. 4 is an explanatory view schematically showing variations in dripping quantity in a nozzle of which the surface of the front end surface is subjected to a liquid-repellency treatment.

First, liquid droplets dripped from a nozzle of which the front end surface is subjected to a liquid-repellency treatment, if normal, become spherical at the middle of the opening of the nozzle front end, as shown in Fig. 4(a), and the weight of liquid droplets reach a prescribed level, they are removed from the nozzle front end and fall down and dripped.

However, when the liquid repellency of the nozzle surface subjected to a liquid-repellent treatment is biased, liquid droplets poured from the nozzle move to a side with a lower liquid repellency, and as shown in Fig. 4 (b), the state is deviated from the center of the nozzle opening. In such a state, since the droplet becomes larger than as compared with the normal case, the quantity of liquid droplets dripped becomes larger than that in the normal case.

Further, when a liquid is poured out of the nozzle, the so-called entrainment of air (i.e. the bubbles are generated and mixed in the liquid) may occur. When such entrainment of air occurs, liquid droplets poured from the nozzle are separated into a plurality of liquid droplets having different liquid quantities as shown in Fig. 4 (c), for example. As these plural droplets are dripped individually or as an integrated droplet, the quantity of dripping may be different from that in the normal case.

On the other hand, when a water repellency treatment is conducted for the front end part of a nozzle, further as shown in Fig. 4(d), if a protruded part that protrudes from the front end surface (a peripheral protruded part (burr) shown in Fig. 4(d)) is present on the outer periphery of the nozzle front end, it becomes possible to prevent variations in dripped quantity as mentioned above.

That is, due to the presence of a protruded part on the outer periphery of the opening of the nozzle, liquid droplets poured from the nozzle do not contact the nozzle front end, and become spherical in a state that contacts only the front end part of the protruded part. Therefore, liquid droplets are inducibly formed on the middle of the opening of the front end of the nozzle, and hence, it is possible to prevent liquid droplets being poured on biased positions of the nozzle front end surface, and also possible to prevent liquid droplets from pouring after being separated into plural droplets due to presence of bubbles or the like in droplets poured. Therefore, biasing or variations of liquid droplets as shown in Figs. 4(b) and (c) does not occur, whereby a prescribed quantity of liquid droplets can be poured and dripped.

In the present embodiment, based on this principle, first, by imparting a prescribed liquid-repellent treatment and liquid-repellent structure to the front end part of the nozzle 10 through which a liquid is poured from the container main body 20, liquid repellency of the front end surface of the nozzle 10 is enhanced.

From the viewpoint of eliminating variations in dripping quantity due to variations in liquid repellency of the nozzle surface or entrainment of air or the like, it is desirable to positively create biased liquid repellency so that droplets are formed on the center of the nozzle 10 without fail. A surface having lower liquid repellency than that of the nozzle surface which is subjected to a liquid-repellent treatment is provided at the center of the nozzle.

Specifically, the nozzle 10 of the present invention can be implemented as the nozzle 10A according to the first embodiment and the nozzle 10B according to the second embodiment shown below (see Figs. 1 and 2).

[First embodiment]

In the nozzle 10A of the first embodiment, the surface of the front end part 11A of the nozzle 10A is fluorinated and roughened by a predetermined method.

First, in the nozzle 10A formed of a plastic molded body made of a non-fluorine-based resin, fluorine atoms are incorporated in a molecular chain of the non-fluorine-based resin constituting the plastic molded body.

Further, the surface of the front end part 11A of the nozzle 10A that is fluorinated as mentioned above can be roughened according to need.

By fluorinating and roughing the front end part 11A of the nozzle 10A, the water repellency of the nozzle 10A can be enhanced (see Figs. 3 and 4), a liquid (eye drop) poured from the container main body 2 is prevented from wetting a large range of the front end part 11A of the nozzle 10A, and by adjusting and setting the inner diameter of the opening 11Aa, the dripping quantity of a liquid poured from the nozzle 10A can be arbitrarily set.

As mentioned above, in the first embodiment, by fluorinating and roughing the front end part 11A of the nozzle 10A, a prescribed liquid-repellent treatment and liquid-repellent structure are imparted to the front end part 11A of the nozzle 10A from which a liquid is poured from the container main body 2.

Regarding the operation principle of the liquid repellency structure by fluorinating and roughing the front end 11A of the nozzle 10A of the present embodiment, and an explanation will be made later with reference to Figs. 7 to 10.

By increasing the liquid repellency of the nozzle 10A in this way, it is possible to pour and drip a liquid in a desired dripping quantity (for example, 10 $\mu$l or less) according to the inner diameter of the opening 11Aa of the nozzle 10A.

Further, the nozzle 10A thus subjected to a liquid-repellent treatment is protected by a cap 20 described later, so that breakage, deterioration or the like of the roughened structure of the front end part 11A of the nozzle 10A do not occur.

As described later, it is preferred that the front end part 11A of the nozzle 10A be fluorinated and that the surface of the front end part 11A be roughened in respect of improving liquid repellency.

However, as long as the front end part 11A of the nozzle 10A is at least fluorinated, liquid repellency can be imparted to the nozzle 10A made of a non-fluorine-based resin. Further, as mentioned later, a plasma treatment (see Fig. 20) in order to impart the front end part 11A to be fluorinated is significantly strong, and hence, fine concavities and convexities are formed on the surface of the front end part 11A of the nozzle 10A by a plasma treatment, whereby the surface is roughened.

Accordingly, in the nozzle 10A according to the present embodiment, it suffices that at least the front end part 11A is fluorinated, and if necessary, it suffices that the surface of the front end part 11A is roughened.

[Second embodiment]

The basic configuration, material, or the like of the nozzle 10B according to the second embodiment are the same as those of the nozzle 10A according to the first embodiment mentioned later.

As for the nozzle 10B according to the second embodiment, the constitution of the nozzle front end part differs from that of the nozzle 10A of the first embodiment.

That is, in the nozzle 10B according to the second embodiment, the front end surface has a first surface 11 Ba positioned on the nozzle center side and a second surface 12Ba continuing to the outer peripheral side of the first surface 11Ba, and the first surface 11Ba and the second surface 12Ba formed of surfaces differing in surface free energy. Specifically, the second surface 12Ba has a high liquid repellency than that of the first surface 11Ba.

More specifically, in the example shown in Fig. 2, the nozzle 10B is configured by combination of a first dripping part 11B and a second dripping part 12B which are formed separately.

As shown in Fig. 2(b), the second dripping part 12B constitutes the main body of the nozzle 10B, and at the center of the front end part of the second dripping part 12B, an opening through which the first dripping part 11B is inserted (through hole) are provided. The surface of the front end part of this second dripping part 12B serves as the second surface 12Ba.

The first dripping part 11B is formed of a hollow cylindrical member which is inserted into and engaged with the through hole at the center of the second surface 12Ba of the second dripping part 12B, and the first dripping part 11B constitutes a nozzle opening through which a liquid stored in the container main body 2 can pass and is dripped. Then, the surface of the front end part of this first dripping part 11B forms the first surface 11Ba.

As described above, in the second embodiment, the second dripping part 12B and the first dripping part 11B are integrated to form the nozzle 10B.

The surfaces of the front end parts of the first dripping part 11B and the second dripping part 12B constitute a first surface 11Ba and a second surface 12Ba, and the first surface 11Ba and the second surface 12Ba having different surface free energies.

If the surface free energy of a solid is larger than the surface free energy of a liquid, the liquid wets easily the solid. On the contrary, if the surface free energy is smaller, the liquid hardly wet the solid, and the solid exhibits liquid repellency.

That is, the liquid repellency changes in accordance with the magnitude of the surface free energy of the solid.

Allowing the surface free energies of the first surface 11Ba and the second surface 12Ba to be different (imparting liquid repellency) is as described above with reference to Figs. 3 and 4, and the operation principle of the liquid-repellent structure will be described later with reference to Figs. 7 to 10.

As described above, in the nozzle 10B according to the second embodiment, the front end part surface thereof has two types of surface configuration, i.e. the first surface 11 Ba positioned on the nozzle center side and the second surface 12Ba continuing the outer surface side of the first surface 11Ba. That is, the front end part surface is configured such that the first surface 11Ba and the second surface 12Ba have different surface free energies, i.e. the second surface 12Ba has higher surface repellency than that of the first surface 11Ba.

In the second embodiment, by using such standard of liquid repellency, regarding the two surfaces constituting the front end part surface of the nozzle 10, i.e. the first surface 11Ba and the second surface 12Ba, the nozzle 10B is constituted such that the liquid repellency of the second surface 12Ba becomes higher than that of the first surface 11Ba by allowing the first surface 11Ba to be a high energy surface and the second surface 12Ba to be a low energy surface.

For example, the first surface 11Ba may be configured to satisfy $\theta_E < 90°$ with respect to a liquid as an object and the second surface 12Ba may be configured to satisfy $\theta_E \geqq 90°$ with respect to a liquid as an object.

More specifically, in the present embodiment, the first surface 11Ba is formed of, for example, a bulk plastic resin (having low liquid repellency) as it is, and the second surface 12Ba is configured to be a surface having higher liquid repellency as in the case of the nozzle 10A of the first embodiment by subjecting the surface of the front end part of the nozzle 10B to a surface treatment (e.g. fluorination or surface roughing treatment) by a predetermined method.

Normally, the surface of a plastic resin that is not subjected to any surface treatment has the above-mentioned "contact angle" of $\theta_E < 90°$ for a liquid such as an eye drop that contains a surfactant or oil, and the liquid repellency is "low" (high energy with high wettability). On the other hand, by subjecting the surface of the resin to a surface treatment such as fluorination or surface roughening, it is possible to modify the surface to have a "high" liquid repellency (low energy with low wettability) where the "contact angle" becomes $\theta_E \geqq 90°$.

By doing this, among the two surfaces constituting the front end surface of the nozzle 10B, the liquid repellency of the first surface 11Ba to be "low" (the surface free energy is high) and the liquid repellency of the second surface 12Ba to be "high" (the surface free energy is low).

Here, as for the second surface 12Ba of which the liquid repellency is allowed to be "high" (the surface free energy is low), as in the case of the nozzle 10A according to the first embodiment, for the surface of the front end part of the nozzle 10B formed of a plastic molded body formed of a non-fluorine-based resin, it can be fluorinated by incorporation of fluorine atoms. Further, the second surface 12Ba of the nozzle 10B thus fluorinated can be surface-roughened according to need.

By fluorinating and roughening the second surface 12Ba of the front end part of the nozzle 10B in the above-mentioned way, by increasing the liquid repellency of the first surface 11Ba on the central side of the nozzle 10B, when a liquid (eye drop) is poured from the container main body 2, it is possible to induce such that liquid droplets are formed only on the first surface 11Ba, whereby it becomes possible to prevent a poured liquid from spreading and wetting a wide range towards the second surface 12Ba.

Therefore, by adjusting and setting the inner diameter of the opening of the nozzle 10B and the surface area and shape of the first surface 11Ba, it is possible to set the quantity of liquid dispensed from the nozzle 10B to an arbitrarily small quantity.

As described above, in the nozzle 10B according to the second embodiment, the surface of the front end part has the first surface 11Ba positioned on the nozzle central side and the second surface 12Ba continuing to the outer peripheral side of the first surface 11Ba, whereby the second surface 12Ba has higher liquid repellency than that of the first surface 11 Ba.

More specifically, as described above, the nozzle 10B is composed of two members, that is, the first dripping unit 11B and the second dripping unit 12B, and the surface of the first dripping part 11B is allowed to be the first surface 11Ba, the surface of the second dripping part 12B is allowed to be the second surface 12Ba and only the second surface 12Ba is subjected to a predetermined water repellency treatment, thereby to allow it to have a higher liquid repellency than that of the first surface 11Ba.

By providing the second surface 12Ba for enhancing the liquid repellency of the nozzle 10B and the first surface 11Ba for guiding liquid droplets to the center of the nozzle 10B, according to the inner diameter of the opening of the nozzle 10B and the surface area or shape of the first surface 11Ba, a liquid in a desired quantity (e.g. 10 μl or less) can be poured and dripped stably without causing variations in dripping quantity.

The nozzle 10B having the first/second surfaces 11Ba and 12Ba as mentioned above is protected by a cap 20 which will be described later, and breakage, deterioration or the like of the droplet processing and droplet structure of the second surface 12Ba of the nozzle 10B can be prevented.

In the nozzle 10B according to the second embodiment as described above, similarly to the case of the first embodiment, it is preferable that the front end part of the nozzle 10B be fluorinated and surface-roughened in view of improvement in liquid repellency.

However, as long as the front end part of the nozzle 10B is at least fluorinated, liquid repellency can be imparted to the nozzle 10B made of a non-fluorine-based resin. As will be described later, the plasma treatment (see Fig. 17) for fluorinating the front end surface is very strong, and fine concavities and convexities are formed on the surface of the front end part of the nozzle 10B by the plasma treatment and the surface is roughened.

Therefore, with regard to the nozzle 10B of the second embodiment, it suffices that the second surface 12Ba be at least fluorinated, and if necessary, the surface of the front end part be further surface-roughened.

[Constitution of the nozzle surface]

Hereinbelow, a specific surface configuration of the nozzle 10B according to the second embodiment mentioned above that has two-step surface structures, i.e. the first surface 11Ba and the second surface 12Ba, will be explained with reference to Figs. 5 and 6.

Fig. 5 and Fig. 6 are explanatory views that schematically show the embodiments of the first/second surfaces of the nozzle front end of the nozzle 10B according to the second embodiment.

In the nozzle 10B according to the second embodiment, as shown in Fig. 5(a), when it comprises the cylindrical first dripping part 11B and the second dripping part 12B that is arranged on the outer periphery of the first dripping part, the front end (first surface 11Ba) of the first dripping part 11B can be protruded from the front end (second surface 12Ba) of the second dripping part 12B.

Due to such a configuration, the first dripping part 11B (first surface 11Ba) is present in a protruded manner on the outer periphery of the opening of the nozzle 10B. Further, since the second surface 12Ba has higher liquid repellency than that of the first surface 11Ba, liquid droplets poured from the nozzle 10B are formed and induced such that they are brought into a state that they do not contact the second dripping part 12B (second surface 12Ba) and contact only the surface (first surface 11Ba) of the protruded surface of the first dripping part 11B.

Even if the second surface 12Ba has variations in liquid repellency, the droplets are not affected by this, and even when air entrainment occurs, the liquid droplets are not separated or dispersed on the side of the second surface 12Ba, and they are always induced so as to be formed at the center of the opening of the nozzle 10B. As a result, reliable and stable pouring and dripping can be conducted without causing biasing or variations in liquid droplets.

Further, as compared with the basic structure as described above, for example, as shown in Fig. 5(b), it is possible to make the cylindrical part constituting the first dripping part 11B (the first surface 11Ba) thicker.

Due to such configuration, it is possible to make the area of the first surface 11Ba that has low liquid repellency (having high wettability) larger, whereby liquid droplets can be induced to the center of the nozzle more reliably, and as compared with the case shown in Fig. 5(a), larger droplets can be formed.

In this case, as shown in Fig. 5(c), for example, the side surface of the first dripping part 11B protruded from the second dripping part 12B can be subjected to a prescribed liquid repellency treatment as in the case of the second surface 12Ba.

Due to such a configuration, liquid droplets are repelled from the protruded side surface of the first dripping part 11B, and as a result, as compared with the case shown in Fig. 5(b), liquid droplets can be repelled and separated from the second surface 12Ba further reliably, thereby allowing liquid droplets to be induced and formed on the center of the nozzle.

As shown in Fig. 5(d), the first dripping part 11B can be configured such that the first surface 11 Ba and the second surface 12Ba become almost "flushed" so that it is prevented from protruding from the front end (second surface 12Ba) of the second dripping part 12B.

In this case, due to high liquid repellency of the second surface 12Ba (low energy surface) and high wettability of the first surface 11Ba (high energy surface), it is possible to inducibly form liquid droplets in a state that they contact only the surface part of the first dripping part 11B (first surface 11Ba) without moving to the second dripping part 12B (second surface 12Ba).

Further, in this case, since the first dripping part 11B is not protruded, a spherical body of a liquid droplet is prevented from spreading widely, and as a result, as compared with a case shown in Fig. 5(a), it is possible to form a smaller liquid droplet with a smaller liquid quantity.

Further, in Figs. 5(a) and (b), the cross section including the nozzle central line of the surface part of the first dripping part 11B (first surface 11Ba) has a rectangular shape. As shown in Fig. 5(e), by allowing the front end of the first dripping part 11B to be tapered, it is possible to allow the first surface 11Ba to be a tapered shape that becomes narrower in the dripping direction.

Due to such a configuration, it is possible to allow the area of the first surface 11Ba that is formed of the front end surface of the first dripping part 11B to be smaller than the cases shown in Figs. 5(a) and (b), and by reducing the adhesiveness between the first surface 11Ba and the liquid droplets, it is possible to allow liquid droplets formed to be smaller with a smaller liquid quantity.

Further, it is possible to allow the area of the first surface 11Ba formed of the front end surface of the first dripping part 11B to be further larger as compared with the cases shown in Figs. 5(a) to (e).

For example, as shown in Fig. 5(f), by forming the front end of the first dripping part 11B so as to spread in the form of a trumpet and by forming the first surface 11Ba in a tapered shape enlarging in the dripping direction, the first surface 11Ba can be made larger and wider.

Due to such a configuration, it is possible to make the area of the first surface 11Ba formed by the front end surface of the first dripping part 11B larger than that in the case of Figs. 5 (a) to (e), and by increasing the adhesive power between the first surface 11Ba and liquid droplets, it becomes possible to hold liquid droplets with a larger liquid quantity and a larger spherical shape, and as a result, it becomes possible to form larger droplets having a larger liquid quantity.

Further, the first/second surfaces 11 Ba and 12 Ba as described above are constituted by two separate parts of the first/second dripping parts 11B and 12B, for example. Other than this, as shown in Fig. 5(g) it is also possible to form the first surface 11Ba projecting into the nozzle together with the second surface 12Ba at the front end part of the second dripping part 12B.

The first surface 11Ba that integrally protrudes from the front end opening of the second dripping part 12B can be formed of burrs that are naturally formed when an opening (through hole) is bored in the second dripping part 12B by means of a drill or the like (see Fig. 4(d)), or can be formed by injection molding.

In this case, after the first surface 11Ba is projectingly formed at the front end part of the second dripping part 12B, in a state where the first surface 11Ba is protected by coating, etc., the front end part of the second dripping part 12B is subjected to a fluorination and surface-roughing treatment mentioned later, whereby the second surface 12Ba can be formed.

Due to such a configuration, not only for a case where the first surface 11Ba is projectingly formed on the front end part of the second dripping part 12B, but also for a case where the first surface 11Ba is not protruded from the second surface 12Ba as shown in Fig. 5(d), it is possible to form the first/second surfaces 11Ba and 12Ba integrally in the second dripping part 12B.

By integrally forming the first/second surfaces 11Ba and 12Ba by using the same parts, it is possible to reduce the number of components or simplify the production process.

Further, when the first dripping part 11B and the second dripping part 12B are formed of separate parts, not only the first dripping part 11B has a tubular body as shown in Figs. 5(a) to 5(g), but also, as shown in Fig. 5(h), for example, it is also possible to use a means with which a liquid is dripped utilizing a capillary phenomenon with a fiber member, a nonwoven fabric, etc. as the first dripping part 11B.

As mentioned above, the first dripping part 11B that constitutes the nozzle 10 according to this embodiment is not limited to a cylindrical body or a tubular body as long as it is capable of dripping from the front end part of the nozzle a prescribed quantity of a liquid in the container main body 2.

Further, as shown in Fig. 6 (a), the first dripping part 11B is prevented from protruding from the front end (the second surface 12 Ba) of the second dripping part 12B, and the first surface 11Ba is formed to have a "chamfered" shape in which it is recessed in a tapered manner inwardly to the second surface 12Ba. This configuration can be formed by inserting the first dripping part 11B of which the inner surface of the end part is chamfered in a tapered way into the second dripping part 12B that forms the second surface 12Ba.

Even in this case, liquid droplets poured from the nozzle 10 do not contact the second dripping part 12B (the second surface 12Ba), and are brought into a state that they contact only the surface of the first dripping part 11B (first surface 11Ba) that is recessed in a tapered manner, whereby liquid droplets can be induced to the center of the opening of the nozzle 10, and reliable pouring and dripping can be conducted.

Further, when the first surface 11Ba is formed into a chamfered shape that tapers inwardly to the second surface 12Ba as mentioned above, as shown in Fig. 6(b), the first surface 11Ba having a chamfered shape can be integrally formed with the second surface 12Ba at the front end part of the second dripping part 12B.

By doing so, it is possible to form the first/second surfaces 11Ba, 12Ba only with the second dripping part 12B, and as a result, it is possible to reduce the number of parts and to simplify the manufacturing process, etc. In particular, the step of inserting the first dripping part 11B and the step of aligning the first/second surfaces 11Ba, 12Ba become unnecessary, and the first surface 11Ba can be formed by only chamfering the inner surface of the end part of the opening for the second dripping part 12B in which the second surface 12Ba is formed in advance, whereby the production process can be significantly simplified and facilitated.

[Operation principle of liquid-repellency structure]

Next, liquid-repellency structure by fluorination and surface roughing provided on the surface of the front surface of the nozzle 10 according to the embodiment of the present invention (the surface of the front end part 11A of the nozzle 10A of the first embodiment and the second surface 12Ba of the nozzle 10B of the second embodiment) and its operation principle will be explained with reference to Figs. 7 to 10.

Here, in order to improve the liquid repellency to the liquid, it is generally conceivable to use a fluorine-containing resin such as polytetrafluoroethylene (PTFE) as the plastic. However, the contact angle of PTFE to water is at most about 115°, and does not exhibit liquid repellency for a liquid containing a substance having a low surface tension such as alcohol or oil. In addition, since the fluorine-containing resin is very expensive and difficult to mold, there is a problem that the application thereof, etc. are extremely limited.

For this reason, it is a subject to improve the liquid repellency of a plastic molded body formed by using a fluorine-free non-fluorine-based resin such as polyolefin or polyester.

As means for enhancing the liquid repellency of the liquid, means for providing a liquid-repellent film on the surface of the nozzle or the like or means for forming concavities and convexities can be considered.

For example, by providing a liquid repellent thin film different from the base material (for example, a film containing a compound or resin containing fluorine, silicon or the like) on the surface, it is possible to improve liquid repellency. However, according to such a method, adhesion to the base material tends to be insufficient, and when the dripping is repeatedly performed, the liquid-repellent thin film or the like is peeled off and falls off, not only the liquid repellency is lost, but also there is a risk that the content liquid is contaminated.

On the other hand, means for providing concavities and convexities on the surface of a nozzle or the like physically imparts liquid repellency by the surface shape, and hence problems regarding a thin film, etc. as mentioned above do not occur.

That is, when a liquid flows on the concavo-convex surface formed on the surface of the nozzle, etc., an air pocket is formed in the concave parts, the contact state between the concavo-convex surface and the liquid becomes a mixed contact state of solid-liquid contact and air-liquid contact. In addition, a gas (air) is a substance having highest liquid repellency. Therefore, by appropriately setting the roughness and denseness of the concavities and convexities, a significantly high liquid repellency can be realized.

However, care should be taken that a liquid is repeatedly flown on the concavo-convex surface, a liquid is gradually stored in the concave part, and the function of the air pocket is gradually lost, and water repellency is gradually lowered.

In the embodiment of the present invention, first, fluorine atoms are incorporated into a molecular chain of a non-fluorine-based resin of a plastic molded body that constitutes the surface of the nozzle 10 (the front end part 11A of the nozzle 10A of the first embodiment and the second dripping part 12B of the nozzle 10B of the second embodiment).

In addition, the surface of the front end part of the nozzle 10 thus fluorinated (the surface of the front end part 11A of the first embodiment and the second surface 12Ba of the second embodiment) is surface-roughed to further form concavo-convex part.

In the nozzle 10B of the second embodiment, the first surface 11Ba having lower liquid repellency than that of the second surface 12Ba is disposed at the center of the roughened nozzle surface. Specifically, in the center of the opening of the second dripping part 12 that is subjected to a liquid-repellent treatment, a tubular and cylindrical first dripping part 11 is inserted and fitted.

In the nozzle 10A of the first embodiment, as part of the surface roughed structure, a circumferential protruded part 13 that protrudes from the front end surface (see Fig. 4(d)) can be provided on the outer periphery of the surface-roughened opening 11a of the nozzle 10.

By increasing the liquid repellency of the nozzle 10 in this way, a desired dripping quantity (e.g. 10 μl or less) of a liquid can be poured and dropped in accordance with the inner diameter of the opening of the front end part of the nozzle 10.

Further, the nozzle 10 that is subjected to a liquid-repellency treatment in this way is protected by a cap 20 mentioned later, whereby breakage, deterioration or the like of the surface-surface-roughened structure at the front end part of the nozzle 10 are prevented from occurring.

Fig. 7 shows a morphology of a roughened surface formed on the surface of the front end part in a plastic molded body constituting the nozzle 10 according the embodiment of the present invention (the front end part 11A of the nozzle 10A of the first embodiment and the second dripping part 12B of the nozzle 10B of the second embodiment 10B).

In this figure, the surface of the front end part is formed of a non-fluorine-based resin. In this surface, a roughened surface 100 formed of minute concavities and convexities is formed (in Fig. 7, the top of the convex part in the roughened surface 100 is denoted by S).

Fluorine atoms are incorporated into a molecular chain of the non-fluorine-based resin forming this roughened surface 100 by a post-treatment. For example, when the molecular chain of the non-fluorine-based resin is represented by $-(CH_2)_n-$, a fluorine atom is incorporated in a part of this molecular chain to form a fluorine-containing moiety such as $-CHF-$ or $-CF_2-$. Such post-processing for incorporating fluorine atoms can be performed by fluorine plasma etching described later (see Fig. 17).

The liquid repellency on the roughened surface 100 mentioned later will be explained with reference to Fig. 8.

As shown in Fig. 8(a), with respect to the contact pattern of the liquid droplet on the roughened surface 100 as described above, in the Cassie mode in which droplets are placed on the roughened surface 100, the concave part in the roughened surface 100 is an air pocket, and the droplets are brought into a state of composite contact of a solid and a gas (air). In such composite contact, the contact radius R at the contact interface of droplets is small, the adhesiveness between liquid droplets and the roughened surface is low, and hence the liquid comes in contact with the air with the highest liquid repellency, whereby high liquid repellency is exhibited. The contact angle of the roughened surface 100 in such Cassie mode is represented by the following theoretical formula (1):

$$\cos\theta^* = (1-\varphi_S)\cos\pi + \varphi_S\cos\theta_E$$
$$= \varphi_S - 1 + \varphi_S\cos\theta_E \quad (1)$$

$\theta_E$: Contact angle
$\theta^*$ : Apparent contact angle
$\varphi_S$: Area ratio (projection area of the solid-liquid interface per unit area)

As can be understood from this theoretical formula (1), as $\varphi_S$ gets small, the apparent contact angle $\theta^*$ gets closer to 180°, whereby ultrahigh liquid repellency is exhibited.

On the other hand, if liquid droplets enter the recess of the roughened surface 100, the liquid droplets are brought into contact only with a solid, not in the above-mentioned composite contact, and is shown by the Wenzel mode. In such Wenzel mode, the contact angle R in the contact interface of the liquid droplets is large, and adhesion power between the liquid droplets and the roughened surface is high. The contact angle of the concavo-convex surface is represented by the following theoretical formula (2):

$$\cos\theta^* = r\cos\theta_E \quad (2)$$

$\theta_E$: Contact angle
$\theta^*$ : Apparent contact angle
r: Concavo-convex degree (= actual contact angle/projected area of liquid droplets)

As can be understood from this theoretical formula (2), the larger r, the closer the apparent contact angle $\theta^*$ to 180°, whereby ultrahigh liquid repellency is exhibited.

As for the liquid repellency, as mentioned above, it is known that liquid repellency is improved in either the Wenzel mode or the Cassie mode. In order to reduce the adhesiveness between the roughened surface 100 and the liquid droplets to allow small quantity of liquid droplets to drip, it is required to maintain stably the Cassie mode, not the Wenzel mode, i.e. the air pocket of the concave part is stably maintained.

That is, in the Wenzel mode, the interface between the liquid phase and the solid phase is large, and as a result, the physical adsorption force acting on the interface is also increased, whereby the contact angle is large and the liquid repellency is achieved, and hence, liquid droplets do not drip or fall easily.

On the other hand, in the Cassie mode, since the interface is small, the adhesiveness which is required to be overcome when liquid droplets drop, the liquid droplets can drip and fall easily, and it is considered that the liquid droplets drop repeatedly many times.

Therefore, in the present embodiment, in order to effectively maintain the contact of the droplets in the above-described Cassie mode, by incorporating fluorine atoms into a molecular chain of the non-fluorine-based resin forming the roughened surface 100 of the front end part of the nozzle 10, liquid repellency is chemically imparted.

That is, if the liquid enters the concave part in the roughened surface 100, the contact pattern of the liquid droplets becomes the Wenzel mode, and as a result, the ultrahigh liquid repellency by the Cassie mode is impaired. In the present embodiment, by incorporating fluorine atoms into a molecular chain of the non-fluorine-containing resin forming the roughened surface 100, it is possible to chemically impart liquid repellency to the rough surface 100, the ultrahigh liquid repellency by the Cassie mode can be stably maintained.

In particular, in the present embodiment, at least part of the roughened surface 100 (e.g. at the top of the convex part or at the bottom of the concave part), a fluorine atom is incorporated in a molecular chain of the non-fluorine-based resin forming this surface in order to exhibit chemical liquid repellency. Therefore, even when the liquid is repeatedly brought into contact with the roughened surface 100, this fluorine atom is not removed, the chemical liquid repellency is stably maintained, and as a result, the ultrahigh liquid repellency in the Cassie mode is not lowered and is maintained at a level as high as that in the initial stage.

Furthermore, instead of forming a film containing fluorine atoms, fluorine atoms are incorporated in a molecular chain of the non-fluorine-based resin on the surface, so that problems such as peeling off or falling off of the fluorine film do not occur at all.

Here, as for the concavo-convex degree of the roughened surface 100 as described above, in order to allow the liquid repellency to be fully exhibited by the Cassie mode, it is preferable that the area ratio $\varphi s$, that is expressed by the area of the top of the convex part S per unit area in the roughened surface 100, be 0.05 or more, preferably 0.08 or more.

Further, in respect of moldability or mechanical strength, the area ratio $\Phi$ is preferably 0.8 or less, in particular 0.5 or less.

Further, the depth d in the roughened surface 100 is preferably 5 to 200 $\mu$m, in particularly 10 to 50 $\mu$m.

Regarding the roughened surface 100, a concavo-convex structure shown in Fig. 9 can be taken.

That is, liquid droplets having a surface tension $\gamma$ and an initial contact angle $\theta$ is, as shown by the following formula (3), supported by the Laplace pressure ($\Delta$p) represented by the concavo-convex apex angle $\alpha$ and the ½ pitch $R_0$ of the concavities and convexities to form an air pocket. That is, when the concavo-convex apex angle $\alpha$ becomes smaller, the ½ pitch $R_0$ becomes smaller, and the concavo-convex structure becomes a pen tip shape, the Laplace pressure becomes larger, whereby liquid droplets hardly enter the concavities and convexities, whereby the liquid repellency is exhibited.

Therefore, as shown in Fig. 9, the larger the arithmetic average roughness Ra representing the amplitude of the concavo-convex structure and the smaller the average length RSm corresponding to the ½ pitch $R_0$, the Laplace pressure is large and the liquid repellency is exhibited. Therefore, Ra/RSm is preferably $50 \times 10^{-3}$ or more, in particular, $200 \times 10^{-3}$ or more.

$$\Delta p = -\gamma \cos(\theta - \alpha)/(R_0 + h\cos\alpha) \qquad (3)$$

In addition, in the present embodiment, formation of the roughened surface 100 composed of the minute concavities and convexities as described above can be generally easily formed by a transfer method using a metal stamper. For example, by a resist method or the like, by heating a stamper having a roughened surface part corresponding to the above-described minute concavities and convexities to an appropriate temperature and pressing it against a predetermined part of the surface of the plastic molded body to transfer the roughened surface part, the roughened surface 100 can be formed on the surface of the front end part of the nozzle 10 made of a plastic molded body. Therefore, the concavo-convex surface of the stamper is formed on the surface of the front part of the nozzle 10 in a state that the concavities and convexities are reversed.

Further, by the roughening treatment by using such a stamper, a thick wall part 15 described later can be formed simultaneously at the outer periphery of the front end part of the nozzle 10 (see Figs. 21 to 26).

In the present embodiment, the roughened surface formed at the front end part of the nozzle 10 is not limited to the concavities and convexities of the roughened surface 100 shown in Fig. 7 or 9. From the viewpoint of stably forming an air pocket, it is preferable that the convex part and the concave part as shown in Fig. 7 be formed in a rectangular shape. For example, when the concave part has a V-shaped configuration, the liquid droplet easily enters the concave part.

Incorporation of the non-fluorine-based resin forming the surface of the nozzle 10 into a molecular chain can be carried out by etching using fluorine plasma.

Here, the fluorine plasma etching can be conducted by using a known method (see Fig. 17 to be described later). For example, by using a $CF_4$ gas, a $SiF_4$ gas or the like to dispose the surface of a plastic molded body forming the roughened surface 100 between a pair of electrodes and applying a high-frequency electric field, plasma of fluorine atoms (atomic fluorine) is generated. By allowing the thus generated plasma to collide with a part forming the roughened surface 100, fluorine atoms are incorporated into a molecular chain of the non-fluorine-based resin forming the surface (roughened surface 100). That is, the resin on the surface is gasified or decomposed, and fluorine atoms are incorporated simultaneously.

Accordingly, ultrafine concavities and convexities are formed in a region where fluorine atoms are incorporated by etching. The arithmetic average surface roughness in these ultrafine concavities and convexities is generally 100 nm or less, and $Ra/RSm \geqq 5 \times 10^{-3}$.

Further, the conditions such as the applied high-frequency voltage and the etching time can be set to appropriate ranges according to the roughness (area ratio $\varphi s$) of the roughened surface 100.

For example, the conditions may be those under which, when the dripped quantity is measured after liquid droplets (eye drop) are repeatedly dripped 100 times in a dripping resistant test to make the front end of the nozzle contaminated, performance of dripped quantity $\leqq 10\ \mu L$ is exhibited. Such conditions may preferably be set in advance by a laboratory test, etc.

Generally, when the element ratio (F/C) of fluorine atoms and carbon per unit area is 40% or more, particularly 50 to 300%, the surface strength is impaired, although it depends on the roughness of the roughened surface 100. In addition, it is possible to ensure stable ultrahigh liquid repellency as described above. The element ratio can be calculated by analyzing the elemental composition on the surface using an X-ray photoelectron spectroscope.

Further, in the present embodiment, the roughened surface 100 formed at the front end part of the nozzle 10 is not limited to the embodiment shown in Fig. 7 or Fig. 9 described above. For example, as shown in Fig. 10, the roughened surface 100 can be formed by a fractal hierarchy structure.

Specifically, as shown in Fig. 10, it is possible to form fine secondary concavities and convexities on the primary concavities and convexities 160 formed by relatively large convex parts 160a and concave parts 160b. In this way, since a droplet 170 is placed on the secondary concavities and convexities, an air pocket (secondary air pocket) is also formed between the liquid droplet 170 and the secondary concavities and convexities. The secondary air pocket between the liquid droplet 170 and the secondary concavities and convexities prevents the entry of the liquid droplet 170 into the concave part 160b of the primary concavities and convexities 160 and it is possible to more effectively prevent the disappearance of the air pocket formed the primary concavities and convexities 160 and the liquid droplet 170. As a result, the state in the Cassie mode can be maintained more stably, whereby liquid repellency can be maintained more stably.

In the roughened surface 100 having the hierarchical structure as described above, the secondary concavities and convexities on the surface part of the primary concavities and convexities 160 have a surface roughness that is enough to allow formation of a secondary air pocket that prevents the liquid droplets on the secondary concavo-convex from entering the concave part 160b of the primary concavities and convexities 160. For example, the ratio of the arithmetic average roughness to the average length, $Ra/RSm$, is preferably $50 \times 10^{-3}$ or more, particularly preferably $200 \times 10^{-3}$ or more.

Further, as the primary concavities and convexities 160, it suffices that they may have the same area ratio $\Phi$ and the depth d of the concavities and convexities as those of the roughened surface 100 having a morphology shown in Fig. 7. As a result, liquid repellency by the Cassie mode can be fully exhibited.

The secondary concavities and convexities are most preferably formed on the entire surface of the primary concavities and convexities 160 from the viewpoint of more effectively preventing the liquid droplet 170 from entering the concave part 160b of the primary concavities and convexities 160. However, it suffices that they are formed at least at the upper end of the convex part 160a of the primary concavities and convexities 160.

The roughened surface 100 having a hierarchical structure as described above can be formed by a method in which minute secondary concavities and convexities are formed on an uneven surface for forming primary concavities and convexities by blasting, etching, etc. and transfer is conducted by using such stamper.

In the present embodiment, in at least part of the primary concavities and convexities 160 on which the secondary concavities and convexities are formed as mentioned above (in particular, a part that forms a top of the convex part 160a or a part that forms a bottom of the concave part 160b of the primary concavities and convexities 160), a fluorine atom is incorporated into a molecular chain of the non-fluorine-based resin forming the surface by plasma etching. In such a region, by etching when a fluorine atom is incorporated, third concavities and convexities that are obtained by further miniaturizing the secondary concavities and convexities are formed. The arithmetic average roughness Ra of the third concavities and convexities is generally 100 nm or less, and Ra satisfies $Ra/RSm \geqq 5 \times 10^{-3}$, as in the case of the ultrafine concavities and convexities formed by etching mentioned later.

The nozzle 10 according to the present embodiment is formed by using a non-fluorine-based resin. As such a non-fluorine-based resin, i.e. a resin containing no fluorine, any thermoplastic resin, thermosetting resin, photocurable resin, etc. can be given as long as it can form the above-mentioned the roughened surface 100 formed of concavities and convexities and can permit incorporation of a fluorine atom into a molecular chain by fluorine plasma etching. An appropriate resin may be selected according to molding conditions, etc. of the nozzle 10. It may be of a multilayer structure.

In general, in the field of liquid containers, olefinic resins typified by polyethylene, polypropylene, copolymers of ethylene or propylene and other olefins, polyesters such as polyethylene terephthalate (PET), polyethylene isophthalate, polyethylene naphthalate and the like are representative as a resin for surface forming.

The nozzle 10 according to the present embodiment as described above can be applied as a nozzle/pouring means of various containers by taking advantage of the long life and excellent liquid repellency of the roughened surface 100. In particular, since falling property and drainability of the liquid is excellent, liquid dripping and presence of residual liquid on the nozzle top surface can be suppressed, it can be effectively used as a nozzle for a container or a wrapping body accommodating various liquid medicine such as the container 1 for instillation of eye drops.

[Cap]

In the container main body 2 including the nozzle 10 that is subjected to a liquid-repellent treatment, a cap 20 is detachably attached. By the cap 20, the nozzle 10 is covered, whereby the inside of the container main body 2 is sealed, and the front end part of the nozzle 10 is protected.

Figs. 11 and 12 are views showing the cap 20 that covers the nozzle 10A according to the first embodiment, and Figs. 13 to 15 are views showing the cap 20 that covers the nozzle 10B according to the second embodiment.

As shown in these figures, the cap 20 is formed of a bottomed cylindrical body that can be attached such that it covers the protruded part of the container main body 2 including the nozzle 10. At the bottom surface of the cylindrical body, a liner 21 for sealing is provided such that it contacts the nozzle 10.

In the cap 20 corresponding to the nozzle 10A of the first embodiment, as shown in Figs. 11 and 12, the liner 21 contacts a side surface 12A of a nozzle 10A.

In the cap 20 corresponding to the nozzle 10B of the second embodiment, as shown in Figs. 13 and 14, the liner 21 contacts the front end surface (first surface 11Ba) of the first dripping part 11B and the side surface part of the second dripping part 12B.

Due to contact or pressure welding of the liner 21 with the front end surface of the side surface part or the front end surface of the first dripping part 11B of the nozzle 10, the nozzle 10 and the container main body 2 are shielded and sealed from the outside, whereby a liquid (eye drop) stored in the container main body 2 is prevented from leaking outside.

On the inner side surface of the cap 20, there is provided a screw structure screwed together with the surface of the projected part of the container main body 2 to which the nozzle 10 is attached. As a result, the cap 20 is detachably attached to the container main body 2 by screwing, and in a state where the cap 20 is attached, the liner 21 is brought into close contact with the side surface part or the front end surface of the first dripping part 11B of the nozzle 10, whereby the inside of the container is sealed.

Here, the cap 20 is formed of a plastic material like the container main body 2 and the nozzle 10. The plastic material forming the cap 20 is not particularly limited, and the cap 20 can be formed by using various thermoplastic resins, e.g. olefinic resins such as polyethylene and polypropylene, or polyester resins represented by polyethylene terephthalate (PET) as in the case of the container main body 2 and the nozzle 10.

In addition, the liner 21 provided on the inner surface of the cap 20 can be formed of a known elastic material, for example, a thermoplastic elastomer such as an ethylene-propylene copolymer elastomer or a styrene elastomer.

Further, the cap 20 can be made of glass or a metal in addition to a plastic material. The cap 20 may be integrally formed with the container main body 2 through a hinge, etc.

The cap 20 according to the present embodiment is configured to cover the protruded part of the container main body 2 including the nozzle 10 without contacting the front end part of the nozzle 10.

The cap 20 is configured so as to cover the protruded part of the container main body 2 including the nozzle 10 in such a manner that, in the cap 20 corresponding to the nozzle 10A according to the first embodiment, the inner surface of the cap 20 including the above-described liner 21 does not contact the front end part 11 of the nozzle 10A, and in the cap 20 corresponding to the nozzle 10B according to the second embodiment, the inner surface of the cap 20 including the liner 21 does not contact the front end surface (the second surface 12Ba) of the second dripping part 12B.

Due to such a configuration, the nozzle 10 can be protected by the cap 20 without contact of the cap 20 with the front end part of the nozzle 10 that is fluorinated and surface-roughened as mentioned above, whereby the liquid-repellent performance and liquid-repellent structure of the front end surface of the nozzle 10 are not deteriorated.

Specifically, as shown in Figs. 1 and 2, in the nozzle 10 according to the present embodiment, the front end part 11A and the second dripping part 12B are formed such that the side surface parts 12A and 12Bb are inclined such that it is tapered towards the front end part.

As shown in Figs. 11 to 14, the liner 21 provided on the inner surface of the cap 20 is formed in a mortar shape corresponding to the tapered shape of the side surfaces 12A and 12Bb of the nozzle 10. Due to such a configuration, in the cap 20, the liner 21 contacts the side surfaces 12A and 12Bb of the nozzle 10, and any part of the cap 20 does not contact the front end part.

As a result, as the liner 21 contacts and is pressed against the side surface side of the nozzle 10, the cap 20 can seal the container main body 2, and the liquid-repellent structure at the front end part of the nozzle 10 is protected without contacting any part.

In the cap 20 corresponding to the nozzle 10A of the first embodiment shown in Fig. 11, in a state that the liner 21 contacts the side surface part 12 of the nozzle 10 attached to the container main body 2, an opening 11a of the front end part 11 of the nozzle 10 is kept open. In this state, the opening 11a is shielded and sealed from the outside by the liner 21, and hence, the liquid is not leaked outside the cap 20 from the opening 11a.

In the cap 20 corresponding to the nozzle 10B of the second embodiment shown in Fig. 13, in a state that the liner 21 contacts the front end part 11 (first surface 11Ba) of the first dripping part of the nozzle 10 attached to the container main body 2, the opening of the nozzle 10 (first dripping part 11) is closed and sealed in a state that it is intercommunicated with the container main body 2. In this state, the opening is sealed by the liner 21, the liquid is not leaked to the outside of the cap 20 from the opening.

In these cases, if the inner diameter of the opening of the nozzle is reduced, the pressure loss increases. Therefore, the liquid never seep out from the opening to the inner surface of the liner 21 of the cap 20 only by the discharge pressure caused by the own weight of the content liquid. However, if the container 1 is crushed by any external force and the inner pressure is increased, the content liquid may seep out.

In this case, the liquid seeped out to the front end part of the nozzle 10 may adhere to the surface of the front end part 11A or the first/second surfaces 11a and 12a. In such a case, adhered liquid may effect adversely when original dripping operation is conducted.

Under such circumstances, the cap 20 can be configured to prevent the liquid from seeping out from the nozzle opening.

For example, as shown in Fig. 12 or 14, the cap 20 is configured to close the front end of the nozzle 10 by pressing the side surface parts 12A and 12Bb continuing from the front end part of the nozzle 10 by the liner 21 arranged on the inner surface of the cap 20.

Specifically, in the cap 20 shown in Figs. 12 and 14, the liner 21 that is in contact with the side surface parts 12A, 12Bb of the nozzle 10 is formed into a mortar shape being inclined more acutely than the tapered shape of the side surface parts 12A, 12Bb, and the side surface parts 12A and 12Bb of the nozzle 10 that are in contact with the liner 21 are pressed towards the center of the nozzle. As a result, due to elasticity of the plastic molded body, the nozzle 10 is deformed towards the inside of the nozzle, whereby the opening (opening 11a or the opening of the first dripping part 11) is closed or blocked.

As a result, in a state where the cap 20 is attached, the opening of the nozzle 10 is closed, and the liquid in the container main body 2 does not seep out from the opening, whereby the problems mentioned above can be solved. The cap 20 may be engaged with and connected to the container main body 2 through a hinge, etc. so that it is not separated from the container main body 2.

In the cap 20 corresponding to the nozzle 10B according to the second embodiment, as shown in Fig. 15, for the nozzle 10B in which the first surface 11Ba shown in Fig. 6(a) and (b) mentioned above is configured in a chamfered shape that is recessed inwardly to the second surface 12Ba in a tapered shape, a needle valve 22 that contacts and is engaged with the tapered chamfered concave part is provided, thereby to close the opening of the nozzle 10.

Due to such configuration, the container main body 2 can be sealed and closed reliably by the needle valve 22 that fits the tapered chamfered concave part without contacting the liquid-repellent treated surface of the front end part of the nozzle 10.

[Method for producing nozzle]

A method of producing the nozzle 10 according to the present embodiment in which the surface of the front end part is fluorinated and roughened as described above will be explained with reference to Figs. 16 to 19.

[Nozzle 10A of the first embodiment]

Fig. 16 is an explanatory view schematically showing the method for producing a nozzle 10A according to the first embodiment of the present invention, in which (a) is a case where common injection molding is used, and (b) is a case where injection compression molding or heat & cool type injection molding is used.

Fig. 17 is an explanatory view schematically showing the method for fluorine plasma etching for roughening the front end part of the nozzle 10 according to the present invention.

As shown in Fig. 16(a), as for the nozzle 10A according to the first embodiment, a nozzle 10 of which the front end part 11 is not fluorinated and roughened can be formed by injection molding, for example.

In this case, as shown in Fig. 16(a)(1), the nozzle 10A can be formed by filling, solidifying, mold-releasing and removing a prescribed molten plastic resin by using a mold for injection molding.

Here, the nozzle 10A can be formed in a predetermined shape and size according to the size and shape of the mold, and the inner diameter of the opening 11a of the nozzle 10A can be set to a desired size, for example, 0.5 mm or less (0.1 mm, 0.2 mm, 0.4 mm, etc.).

Thereafter, as shown in Fig. 16(a)(2), prescribed concavities and convexities can be formed by pressing a prescribed stamper against the surface of the front end part 11A of the nozzle 10A.

Further, as shown in Fig. 16(a)(3), plasma etching is conducted for the surface of the front end part 11A of the nozzle 10A.

The fluorine plasma etching shown in Fig. 16(a)(3) is conducted by a method shown in Fig. 17, for example. That is, one electrode 200 is fixed to the front end part 11A of the nozzle 10A and the other electrode 201 is opposed such that the front end part 11 is placed therebetween, and a high-frequency electric field is applied while flowing a fluorine-containing gas between these electrodes.

By the methods mentioned above, the roughened surface 100 shown in Figs. 7 to 10 mentioned above can be formed and the front end part 11 of the nozzle 10 can be fluorinated and roughened.

As a result, the nozzle 10A according to the first embodiment of the present invention is completed.

Further, by using special injection compression molding or heat & cool type injection molding instead of common injection molding as shown in Fig. 16(a), the nozzle 10 having predetermined concavities and convexities on the surface of the front end part 11 can be formed by integral molding.

By using special injection compression molding or heat & cool type injection molding technology, it becomes possible to subject to a desired part of a molded article to fine concavities and convexities-forming treatment and surface roughening treatment in the molding step. As shown in Fig. 16(b)(1), the molding step of the nozzle 10A and the surface roughing of the front end part 11A can be conducted as a single step. That is, as shown in Fig. 16(b)(2), the concavities and convexities-forming treatment and surface roughening treatment by using a stamper, etc. shown in Fig. 16(a)(2) can be omitted.

Thereafter, as shown in Fig. 16(b)(3), by conducting fluorine plasma etching for the surface of the front end part 11 of the nozzle 10 (see Fig. 17), the fluorinating and surface-roughening treatment are completed.

[Nozzle 10B of the second embodiment]

Fig. 18 is an explanatory view schematically showing the method for producing the nozzle 10B according to the second embodiment of the present invention, in which (a) is a case where common injection molding is used; and (b) is a case where injection compression molding or heat & cool type injection molding is used.

Fig. 19 is an explanatory view schematically showing the method for producing the nozzle shown in Fig. 18(a), showing a case where, in the production method shown in Fig. 18(a), a first surface 11Ba with a chamfered shape is formed on the front end opening of the second dripping part 12B without using the first dripping part 11B.

The method for producing the nozzle 10B according to the second embodiment shown in these figures are basically almost the same as the method for producing the nozzle 10A of the first embodiment mentioned above (Figs. 16 and 17).

However, in the case of the nozzle 10B according to the second embodiment, the second dripping part 12B constituting the nozzle main body and the first dripping part 11B inserted into and engaged with the front end of the second dripping part 12B are separately configured, and hence they are separately produced.

The second dripping part 12B that constitutes the nozzle main body can be produced by the same production method as that of the nozzle 10A of the first embodiment mentioned above.

That is, as shown in Fig. 18 (a), with respect to the second dripping part 12B, first, a second dripping part 12 of which the surface of the front end part is not fluorinated and roughened is formed by injection molding, for example. In this case, as shown in Fig. 18(a)(1), the second dripping part 12B can be formed by filling, solidifying, mold-releasing and removing a prescribed molten plastic resin by using a mold for injection molding.

In accordance with the dimension or shape of the mold, the nozzle 10B (the second dripping part 12B) can be formed into a prescribed shape and to have a prescribed dimension. The nozzle 10B can be formed to have an inner diameter that allows the first dripping part 11B serving as the final opening of the nozzle 10B to be inserted and engaged. Specifically, the nozzle 10B can be formed such that it has an outer diameter that is almost similar to or slightly larger than the outer diameter of the first dripping part 11B.

Further, although not particularly shown, the first dripping part 11B can be produced in a prescribed shape with a prescribed dimension by using injection molding, for example, as in the case of the production of the second dripping part 12B.

Since the opening (inner diameter) of the first dripping part 11B serves as the final opening of the nozzle 10B, it is possible to set the inner diameter thereof into a desired size, for example, 0.5 mm or less (0.1 mm , 0.2 mm, 0.4 mm, etc.).

Thereafter, as shown in Fig. 18(a)(2), by pressing a prescribed stamper against the front end part surface of the second dripping part 12B to form prescribed concavities and convexities, whereby the highly liquid-repellent second surface 12Ba (low energy surface) can be formed.

Further, as shown in Fig. 18(a)(3), fluorine plasma etching is conducted for this second surface 12Ba.

The fluorine plasma etching shown in Fig. 18 (a)(3) is performed in the same manner as in the case of the nozzle 10A of the first embodiment described above. For example, by using the method shown in Fig. 17, one electrode 200 is fixed in the Vicinity of the front end part of the second dripping part 12B, and the other electrode 201 is placed such that it opposes to the electrode 200 so that the surface of the front end part (second surface 12Ba) is arranged therebetween, and a high-frequency electric field is applied while flowing a fluorine-containing gas between these electrodes.

By the above procedures, the roughened surface 100 shown in Figs. 7 to 10 mentioned above can be formed, and the front end part of the second dripping part 12B (second surface 12Ba) serving as the main body of the nozzle 10B can be fluorinated and roughened.

Thereafter, as shown in Fig. 18(a)(4), into a through hole at the center of the front end part of the second dripping part 12B that is fluorinated and roughened, the first dripping part 11B produced in a separate step can be inserted and engaged.

As a result, the nozzle 10B according to the second embodiment is completed.

Further, as for the nozzle 10B according to the second embodiment, as in the case of the nozzle 10A of the first embodiment, by using special injection compression molding or heat & cool type injection molding instead of common injection molding as shown in Fig. 18(a), the second dripping part 12 provided with prescribed concavities and convexities on the front end part surface can be formed by integral molding.

By using special injection compression molding or heat & cool type injection molding technology, it becomes possible to subject to a desired part of a molded article to fine concavities and convexities-forming treatment and surface roughening treatment in the molding step. As shown in Fig. 18(b)(1), the molding step of the second dripping part 12B and the surface roughing of the front end part (forming step of second surface 12Ba) can be conducted as a single step, and one step can be omitted. That is, as shown in Fig. 18(b)(2), the concavities and convexities-forming treatment and surface roughening treatment by using a stamper, etc. shown in Fig. 18(a)(2) can be omitted.

Thereafter, as shown in Fig. 18(b)(3) and (4), by conducting a fluorinating and surface-roughening treatment for the surface of the front end part of the nozzle 10B by plasma etching (see Fig. 17), and by allowing the first dripping part 11B produced in a separate step to be inserted and engaged with the second dripping part 12B, the nozzle 10B is completed.

Regarding the nozzle 10B according to the second embodiment, as shown in Fig. 19, when a first chamfered first surface 11Ba is formed at the front end part opening of the second dripping part 12B without using the first dripping part 11B, as shown in of Fig. 19(1), by using a mold for injection molding corresponding to the outer diameter of the chamfered concave part, the second dripping part 12B having a tapered concave part at the front end part of the opening can be formed (see Fig. 19(2)).

Thereafter, as shown in Figs. 19(3) and (4), a concavities and convexities-forming treatment and a surface roughening treatment by using a stamper, etc. and a fluorinating and surface roughing treatment by using fluorine plasma etching are conducted, whereby the nozzle 10B is completed.

In the roughening/fluorinating treatment, the tapered chamfered concave molded part is masked so as not to be roughened and fluorinated. After roughening and fluorinating the front end part of the nozzle 10B to form the second surface 12Ba, the inner surface of the opening of the second surface 12Ba is chamfered, whereby the tapered first surface 11Ba can also be formed. In this case, the above-mentioned masking treatment becomes unnecessary.

[Bulwark]

Subsequently, an explanation will be made on a case where a bulwark 14 is provided at the front end part of the nozzle 10A according to the first embodiment of the present invention.

In the nozzle 10A according to the first embodiment, on the front end surface of which the liquid repellency is improved by subjecting the front end part 11 to a fluorine plasma treatment or a surface roughing treatment as mentioned above, the bulwark 14 can be further provided.

Fig. 20 is a plan view and a cross-sectional view taken along the line A-A thereof of the front end part when a bulwark 14 is provided on the periphery of the opening of the nozzle 10A, in which (a) shows a case where a bulwark 14 is provided on the periphery of the opening, and (b) shows a case where a bulwark 14 is not provided.

As mentioned above, in the fluorinated and surface-roughened front end part 11 of the nozzle 10A according to the first embodiment, since high liquid repellency is imparted, liquid droplets poured from the container main body 2 can easily fall and drip from the opening 11Aa of the front end part of the nozzle 10A. Further, by surface-roughening of the front end part 11A of the nozzle 10A, the concavo-convex structure obtained by surface roughening and the opening 11Aa of the nozzle 10A may be spatially continued and intercommunicated as shown in Fig. 20(b).

In such a case, part of the liquid droplets poured from the container main body 2 may enter and soak into the concavo-convex structure that is spatially intercommunicated with the opening 11a.

If the content liquid enters and soaks into the concavo-convex structure in this way, there is a possibility that the liquid-repellent performance by the concavo-convex structure is lowered or the liquid remains at the front end part 11A (top surface) of the nozzle 10A, and as a result, the small quantity dripping performance and liquid dripping prevention performance of the nozzle 10A may be deteriorated.

As shown in Fig. 20(a), at the front end part 11A of the nozzle 10A, the bulwark 14 is vertically provided such that it surrounds the periphery of the opening 11Aa.

By the provision of the bulwark 14, intercommunication of the concavo-convex structure of the roughened front end part 11A and the opening 11Aa is shielded and stopped, as a result, liquid droplets poured from the opening 11Aa are prevented from entering and soaking into the concavo-convex structure can be prevented. As a result, lowering in liquid-repellent performance of the roughened front end part 11A and presence of residual liquid on the front end part 11A can be prevented, whereby the small-quantity dripping performance and liquid dripping prevention performance of the nozzle 10A can be maintained for a long period of time.

As shown in Fig. 20(a), such bulwark 14 can be formed of a hierarchical concavo-convex structure (see Figs. 7 to 10) formed on the periphery of the opening 11Aa of the front end part 11A, and can be formed of a circumferential protruded part (see Fig. 4(d)) that is formed in a protruded manner in the opening 11Aa.

[Thick wall part]

When the liquid is dropped in a state where the container is inclined, the liquid droplets poured from the opening is discharged outside the nozzle after flowing along the nozzle front parts 11A and 12Ba (pouring mode).

At that time, liquid dripping phenomenon that liquid droplets move to the nozzle side surfaces 12A and 12B and contaminate the nozzle and the container body problematically occurs.

For the nozzle 10 (10A, 10B) for which the front end part is subjected to a liquid-repellent treatment mentioned above, by providing a thick wall part 15, liquid dripping phenomenon at the time of pouring can be prevented.

Figs. 21 and 22 are each a partial cross-sectional view of the nozzle in which a thick wall part 15 is provided on the outer periphery of the front end part of the nozzle 10 according to the first embodiment, and Fig. 21 shows a case of the nozzle 10A according to the first embodiment and Fig. 22 shows a case of the nozzle 10B according to the second embodiment.

In these Figs. 21 and 22, (a) shows a case where the thick wall part 15 is overhung relative to the top surface of the nozzle front end part, (b) shows a case where the thick wall part 15 is slanted relative to the top surface of the nozzle front end part, and (c) shows a case where no thick wall part 15 is provided in the nozzle.

As shown in (a) and (b) of Figs. 21 and 22, the thick wall part 15 is a part that protrudes from the outer peripheral edge of the front end part of the nozzle 10 to the outside. Due to the provision of the thick wall part 15, drainability of a liquid that moves to the outer peripheral edge of the front end part, i.e. separability between the liquid droplets falling from the outer periphery of the nozzle 10 and the liquid remaining in the nozzle 10, can be improved. As a result, the liquid is prevented from dripping to the side surface side continuing the front end part of the nozzle 10, and in cooperation with the high liquid repellency of the front end part, occurrence of dripping of liquid droplets poured from the opening of the nozzle 10 can be suppressed or prevented.

[Mechanism of liquid drainability]

As for the mechanism of the liquid drainability of the thick wall part 15, an explanation will be made with reference to Figs. 23 and 24.

Fig. 23 is a cross-sectional view of an essential part for explaining the drainability when a slanted thick wall part 15 is provided on the outer periphery of the front end part of the nozzle 10. Fig. 24 is a cross-sectional view of an essential part of the nozzle when an overhung thick wall part 15 is provided on the outer periphery of the front end part of the nozzle 10.

The thick wall part 15 shown in these figures is formed as follows: When the front end part (top surface) of the nozzle 10 is heat-pressed, for example, the resin at the surface layer of the front end part is molten and a part of the molten resin is extruded radially outward from the outer peripheral edge of the tip part and is solidified.

Further, as shown in Fig. 23, for example, the shape of the thick wall part 15 may be in a shape in which the front end thereof protrudes at an acute angle (slanted shape), or in a shape in which the front end thereof protrudes in the form of droplets as shown in Fig. 24 (overhang shape).

Due to the provision of such thick wall part 15, liquid drainability at the outer peripheral edge of the nozzle 10 of content liquid poured out from the opening can be improved, whereby dripping of the content liquid from the outer periphery of the nozzle 10 can be effectively suppressed. The mechanism is explained below.

[Slant mode]

As shown in Fig. 23, in a case where the thick wall part 15 is protruded at an acute angle such that it is substantially flushed with the top surface (the surface of the front end part) of the nozzle 10 (slant mode), when the liquid advancing at a contact angle $\theta_E$ reaches the outer peripheral edge (edge part) of the front end part (see Fig. 23(a)), when the angle formed by the travelling surface of the liquid (the top surface of the front end part) and the outer surface of the edge part is taken as $\alpha$, the liquid stays in the edge part until the advancing angle $\theta^*$ (the critical contact angle of the edge part) becomes $\theta^* = \theta_E + (\pi - \alpha)$ (see Fig. 23(b)).

This is a phenomenon known as the pinning effect in respect of the relationship between the surface tension of the liquid and the contact angle. However, as shown in Fig. 23, if the thick wall part 15 is formed such that the front end part thereof protrudes at an acute angle ($\alpha < 90°$) (slant mode), the advance angle is increased due to the pinning effect, and the content liquid tends to stay in the thick wall part 15 due to the surface tension.

As a result, the drainability of the liquid that moves to the outer peripheral side of the nozzle front end part, i.e. separability of liquid droplets falling from the outer edge of the nozzle 10 and the liquid remains on the top surface side of the nozzle 10 can be improved, whereby dripping of the liquid poured from the nozzle 10 to the nozzle surface side can be suppressed.

In the example shown in Fig. 23, the upper surface of the thick wall part 15 is flushed with the top surface (the surface of the front end part) of the nozzle 10, but when the thick wall part 15 is formed so that the front end thereof has a shape in which the front end protrudes at an acute angle, although not particularly shown, the thick wall part 15 may be formed such that the upper surface thereof is inclined (slanted) linearly or in a curved way with respect to the top surface of the nozzle 10.

[Overhang mode]

Next, as shown in Fig. 24, in the case where the front end of the thick wall part 15 protrudes in the form of droplets so that the traveling surface of the content liquid curves downward (overhangs) in an arc shape (overhang mode), the content liquid which has flown to the root side beyond the lowest point of the thick wall part 15 remains in the thick wall part 15 at a critical contact angle $\theta_E$ due to the surface tension.

At this time, when the angle formed by the tangent line L with the thick wall part 15 at its end and the top surface (front end part) of the nozzle 10 is taken as $\alpha$, the advancing angle $\theta^*$ (the critical contact angle of the edge part) becomes $\theta^* = \theta_E + (2\pi - \alpha)$, and the content liquid does not fall off since it is supported by a large apparent surface tension at the edge part.

When the content liquid drips, large droplets that cannot be supported by surface tension any longer are separated and fall, and the liquid is separated from the outer edge of the nozzle 10 and drops without dripping to the side surface side of the nozzle 10.

Therefore, in this overhung mode, the drainability of the liquid that moves to the outer edge side of the front end part, i.e. separability of the liquid droplets falling from the outer peripheral edge of the nozzle 10 and the liquid remaining on the top surface of the nozzle 10 can be improved, whereby the liquid poured from the nozzle 10 is suppressed from dripping to the nozzle surface side.

As described above, in the present embodiment, it is possible to form, by heat pressing or the like, the thick wall part 15 on the nozzle 10 that is molded into a predetermined shape by injection molding or the like, and by apparently increasing the surface tension of the liquid described by the pinning effect, it becomes possible to allow a residual liquid of the content liquid poured out of the container to be accumulated in the thick wall part 15 easily, thereby improving the liquid drainability.

As a result, it is possible to improve the drainability of the liquid that moves to the outer peripheral edge side of the front end part of the nozzle 10, and, in combination with high liquid-repellent performance (liquid droplet falling property) of the fluorinated and surface-roughened front end part, it is possible to prevent effectively the liquid poured from the nozzle 10 from dripping to the nozzle side surface side.

[Method for producing thick wall part]

Subsequently, a method for producing the thick wall part 15 provided at the front end part of the nozzle 10 (10A, 10B) will be explained with reference to Fig. 25.

Fig. 25 is an explanatory view schematically showing the method for producing the thick wall part 15 by heat pressing in the nozzle 10 (10A, 10B) according to the present invention, in which (a) shows a state prior to heat pressing in which the opening (discharge port) of the nozzle is made large in advance such that it is not blocked by heat pressing; (b) shows a state after heat pressing; and (c) shows a state in which the opening of the nozzle is blocked by heat pressing.

As shown in Fig. 25(a), the thick wall part 15 provided at the nozzle 10 can be formed by pressing a hot plate P for conducting heat pressing against the surface of the front end part, followed by heating and pressurizing, whereby the thick wall part 15 protruding from the outer peripheral edge of the nozzle 10 to the outside of the radial direction can be formed.

The shape, size, etc. of the thick wall part 15 formed by heat pressing can be determined by appropriately adjusting the temperature of a hot plate P when pressing the hot plate P when heat pressing the front end part of the nozzle 10, the pressing force of pressing the hot plate P, the time for which the hot plate P, etc., whereby the desired thick wall part 15 can be formed.

The heat pressing for forming the thick wall part 15 can be conducted simultaneously with a step of forming prescribed concavities and convexities by a stamper on the surface of the front end part of the nozzle 10 shown in Fig. 16 and Fig. 18(a)(2).

Specifically, the stamper for forming concavities and convexities on the front end part of the nozzle 10 (see Fig. 16 and Fig. 18(a)(2)) is formed of the hot plate P shown in Fig. 25(a), and concavities and convexities are formed on the top surface of the front end part and, simultaneously, the thick wall part 15 can be formed at the outer peripheral edge of the front end part.

Further, when the thick wall part 15 is formed by heat pressing, it is preferred that the size and shape of the resin after the resin is molten and swollen be predicted and assumed and that the nozzle length and the nozzle opening be designed larger for the nozzle 10 prior to being subjected to heat pressing.

In the case of the nozzle 10 for small-quantity dripping of a container for instillation of eye drops, the opening for pouring liquid droplets is small, and the opening (discharge port) may become narrow or blocked by heat pressing (see Fig. 25(c)).

As shown in Fig. 25(a), by forming the opening of the nozzle 10 larger in advance in order that the opening (discharge port) is not blocked by heat pressing, the opening can have a prescribed size and length even if the thick wall part 15 is formed by heat pressing (see Fig. 25(b)).

In addition, since the thick wall part 15 can be formed by heat pressing, when producing the nozzle 10, it is not required to modify the existent mold and also it is not required to taken into consideration disadvantages such as deformation when taking out from the mold. As a result, the cost incurred for molds can be suppressed to low.

As mentioned above, by the nozzle 10 according to the embodiment of the present invention, the liquid repellency at the front end of the nozzle 10 can be improved and maintained by subjecting the front end part 10 to a fluorine plasma treatment or a surface roughening treatment.

In the nozzle 10A according to the first embodiment of the present invention, the plastic molded body constituting the nozzle main body is formed of a non-fluorine-based resin such as polyolefin or polyester, but on the surface of the front end part 11A, fluorine atoms are incorporated into a molecular chain of the resin

On the surface of the fluorinated front end part 11A, a roughened surface composed of fine concavities and convexities is formed, according to need.

When the liquid is poured from the inside of the container, the front end part 11A of the fluorinated and roughened nozzle 10A has further high liquid repellency due to improvement in liquid repellency by fluorine atoms and existence of an air pocket because of a roughened surface composed of concavities and convexities (gas-liquid contact).

In the nozzle 10A to which liquid repellency is imparted as mentioned above, falling property of liquid droplets is improved by liquid repellency of the top surface of the front end part 11A, and presence of residual liquid on the top surface of the nozzle can be suppressed or prevented.

Further, by forming the bulwark 14 on the front end part 11A, it is possible to prevent soaking of liquid droplets to the top surface of the front end part 11A, whereby deterioration of liquid repellency can be improved, and the small-quantity dripping performance and liquid dripping prevention performance of the nozzle 10A can be maintained for a long period of time.

Furthermore, by forming the thick wall part 15 on the outer peripheral edge of the front end part 11 of the nozzle 10, it is possible to improve the drainability of poured liquid droplets, and in combination with high liquid repellency (falling property of liquid droplets) by fluorinating and surface roughing, occurrence of liquid dripping can be suppressed or prevented.

In the nozzle 10B according to the second embodiment of the present invention, the front end surface of the nozzle 10B is constituted by a plurality of surfaces having different surface free energies, i.e. a first surface 11Ba and a second surface 12Ba, and by allowing the first surface 11Ba positioned at the center of the nozzle to be a high-energy surface with low liquid repellency and the second surface 12Ba positioned therearound to be a low-energy surface with high liquid repellency, reduction in amount of dropped liquid droplets is realized while inducing liquid droplets such that they are formed at the center of the opening of the nozzle 10B.

As a result, stable small-quantity dripping can be reliably conducted without causing deterioration, etc. in dripping performance or causing variations in dripping quantity even if repeatedly used, whereby a nozzle preferable for use in a container for instillation of eye drops can be realized.

Further, in the nozzle 10B to which liquid repellency is imparted, liquid falling property is improved by liquid repellency at the top surface of the front end part, and presence of residual liquid on the top surface of the nozzle can be suppressed or prevented.

Further, by forming the thick wall part 15 on the outer peripheral edge of the front end part of the nozzle 10B, the drainability of the liquid droplets poured can be improved, and in combination with high liquid-repellent performance (falling property of liquid droplets) by fluorinating and surface roughing, occurrence of liquid dripping can be suppressed or prevented.

As mentioned above, in the nozzle 10B according to the second embodiment, by imparting a prescribed liquid-repellent treatment and a liquid-repellent structure to the front end part of the nozzle from which the liquid is poured, the surface of the front end part of the nozzle 10B, i.e. the second surface 12Ba serving as a low-energy surface is provided.

In addition, from the viewpoint of eliminating variations in droplet quantity due to biased liquid repellency of the nozzle surface or entrainment of air, by positively creating a biased liquid repellency to induce the liquid droplets are always formed at the center of the nozzle 10B, a surface having a lower liquid repellency than that of the second surface 12Ba that is subjected to a liquid-repellent treatment, i.e. a first surface 11Ba serving as a high energy surface, is provided at the nozzle center.

As a result, the first surface 11Ba serving as a high-energy surface is present on the outer periphery of the opening of the nozzle 10B, and around it, there is formed the second surface 12Ba that serves as a low energy surface having higher water repellency than the first surface 11Ba continues, the liquid droplet poured from the nozzle 10B is positively repelled from the second surface 12Ba and positively adsorbed to the first surface 11Ba, and the liquid droplets are formed and induced at the nozzle center in a state of being adsorbed and contacted only with the first surface 11Ba.

Therefore, even if the second surface 12Ba has variations in liquid repellency, or even if air entrapping is present in liquid droplets to be poured, the droplets are always guided so as to be formed at the center of the opening of the nozzle 10B without being biased to the second surface side 12Ba or dispersed.

Accordingly, in the nozzle 10B according to the second embodiment, reliable and stable pouring and dripping can be conducted without causing biasing or variations of liquid droplets.

In addition, by applying a fluorine plasma treatment or a surface roughening treatment to the second surface 12Ba which serves as a low energy surface having high liquid repellency, it is possible to improve and maintain the liquid repellency of the nozzle front end surface. That is, the plastic molded body constituting the second surface 12Ba of the nozzle 10B is formed of a non-fluorine-based resin such as polyolefin or polyester, but in the part constituting the second surface 12Ba, fluorine atoms are incorporated into a molecular chain of a non-fluorine-based resin. Further, in the fluorinated second surface 12Ba that is fluorinated, a roughened surface composed of fine concavities and convexities is formed according to need.

In the second surface 12Ba of the fluorinated and surface-roughened nozzle 10B, when a liquid is poured from the inside of the container, due to improved liquid repellency and presence of an air pocket due to the presence of a roughened surface formed of fine concavities and convexities (gas-liquid contact), further high liquid repellency is ensured. As a result, the poured liquid droplets are induced such that they are formed on the first surface 11Ba at the center of the nozzle without adhering to the second surface 12Ba.

With respect to the fluorination of the front end part of the nozzle 10 according to the present invention, fluorine atoms are incorporated into a molecular chain of the non-fluorine-based resin constituting the surface of the front end part of the nozzle, and hence, fluorine atoms are stably present on the surface of the nozzle front end part without falling off. Therefore, even when the liquid is repeatedly poured, the liquid repellency is not impaired.

That is, the nozzle 10 of which the front end part is fluorinated and roughened, excellent liquid repellency can be maintained for a long period of time, and if the liquid repeatedly contacts, the liquid repellency as high as that of the initial stage is maintained, and as a result, small-quantity dripping becomes possible.

Accordingly, by setting the inner diameter of the nozzle 10 and the surface area or shape of the first surface 11Ba to be a prescribed value, the dripping quantity of the liquid (eye drop) poured from the nozzle 10 can be an arbitrary value, e.g. 10 μl or less. As a result, the dripping quantity can be small and optimized, whereby the eye drop quantity and the dripping quantity that are optimum to the eyes of a human being can be realized.

The front end part of the fluorinated and roughened nozzle 10 is protected by the cap 20. In this case, the inner surface of the cap 20 does not abut and contact the fluorinated and roughened surface of the nozzle 10, and the inside of the container is protected in a sealed state with the nozzle 10 being covered by the cap 20 in a state that the cap 20 does not contact the nozzle surface.

Therefore, even if the cap 20 is repeatedly attached and detached, the fluorinating/roughening performance of the front end part of the nozzle 10 is not impaired, and until the liquid (eye drop) in the container is lost, the small amount dripping performance of the nozzle 10 can be exhibited.

As mentioned above, by the nozzle 10 according to this embodiment, not only reduction in dripping quantity in the eye drop container 1 can be realized, but also liquid dripping or presence of residual liquid on the top surface of the nozzle can be prevented.

Further, by reliably protecting the front end part of the nozzle 10 with the cap 20 while taking care not to deteriorate the functions thereof, it is possible to stably maintain the performance of the nozzle according to the present invention, and preferable instillation operation can be conducted until eye drops in the instillation container for eye drops 1 are run out.

EXAMPLES

Examples of the nozzle according to the present invention will be explained below.

Here, the present invention will be explained more detail with reference to the examples below, however it should be understood that the present invention shall not be limited at all by the examples below.

[First embodiment]

First, examples of a nozzle 10A according to the first embodiment of the present invention will be explained.

Examples 1 to 3 according to the present invention and Comparative Examples 1 to 3 are shown in the following Table 1.

[Table 1]

| | Molding conditions | | | | Results of evaluation | | | | | | | |
|---|---|---|---|---|---|---|---|---|---|---|---|---|
| | Diameter of opening | Ni stamper | | Plasma treatment | Shape of 凹凸 | | | Fluorine content | Small-quantity dripping performance | Dripping quantity control performance | Dripping durability performance | Overall evaluation |
| | | Secondary 凹凸 | Hierarchical 凹凸 | | Primary 凹凸 | Secondary 凹凸 | Tertiary 凹凸 | | | | | |
| | Mm | | | | φs | Ra/RSm | Ra/RSm | F/C | μL | $r^2$ | μL | |
| Example 1 | φ0.1 | × | × | ○ | - | - | $7.5 \times 10^{-3}$ | 74% | ○(1) | 0(0.92) | ○(1) | ○ |
| | φ0.2 | | | | | | | | ○(2) | | ○(2) | |
| | φ0.3 | | | | | | | | ○(4) | | ○(4) | |
| | φ0.4 | | | | | | | | 0(8) | | 0(8) | |
| Example 2 | φ0.1 | × | ○ | ○ | 0.1 | $264 \times 10^{-3}$ | $7.5 \times 10^{-3}$ | 74% | ○(1) | 0(0.83) | ○(1) | ○ |
| | φ0.2 | | | | | | | | ○(1) | | 0(2) | |
| | φ0.3 | | | | | | | | ○(3) | | ○(4) | |
| | φ0.4 | | | | | | | | ○(7) | | ○(8) | |
| Example 3 | φ0.1 | ○ | × | ○ | - | $264 \times 10^{-3}$ | $7.5 \times 10^{-3}$ | 74% | ○(1) | 0(0.83) | ○(1) | ○ |
| | φ0.2 | | | | | | | | 0(3) | | 0(2) | |
| | φ0.3 | | | | | | | | ○(4) | | ○(4) | |
| | φ0.4 | | | | | | | | 0(4) | | 0(8) | |
| Comp. Ex. 1 | φ0.1 | × | × | × | - | - | - | 0% | ×(29) | 0(0.87) | ×(29) | × |
| | φ0.2 | | | | | | | | x(44) | | ×(44) | |
| | φ0.3 | | | | | | | | x(56) | | ×(56) | |
| | φ0.4 | | | | | | | | ×(56) | | ×(56) | |

(continued)

| | | Molding conditions | | | | Results of evaluation | | | | | | | | |
|---|---|---|---|---|---|---|---|---|---|---|---|---|---|---|
| | Diameter of opening | Ni stamper | | Plasma treatment | Shape of 凹凸 | | | Fluorine content | Small-quantity dripping performance | Dripping quantity control performance | Dripping durability performance | Overall evaluation |
| | | Secondary 凹凸 | Hierarchical 凹凸 | | Primary 凹凸 | Secondary 凹凸 | Tertiary 凹凸 | F/C | μL | $r^2$ | μL | |
| | Mm | | | | φs | Ra/RSm | Ra/RSm | | | | | |
| Comp. Ex. 2 | φ0.1 | × | ○ | × | 0.1 | $264 \times 10^{-3}$ | - | 0% | ○(1) | 0(0.60) | ×(30) | × |
| | φ0.2 | | | | | | | | ○(1) | | ×(45) | |
| | φ0.3 | | | | | | | | ○(1) | | x(56) | |
| | φ0.4 | | | | | | | | ○(4) | | ×(57) | |
| Comp. Ex. 3 | φ0.1 | ○ | × | × | - | $264 \times 10^{-3}$ | - | 0% | ○(1) | 0(0.90) | x(29) | × |
| | φ0.2 | | | | | | | | ○(1) | | × (42) | |
| | φ0.3 | | | | | | | | 0(3) | | ×(55) | |
| | φ0.4 | | | | | | | | ○(4) | | ×(57) | |

Examples 1 to 3 and Comparative Examples 1 to 3 shown in Table 1 were tested under the following condition:

(1) Test sample

§ Nozzle main body

- Material

  - Low density polyethylene

    - Grade: LJ8041

- Size: 10 mm in diameter x 10 mm in length
- Opening size: 0.1, 0.2, 0.3, and 0.4 mm
- Method of production of nozzle main body

  - A nozzle with the above-mentioned opening was obtained by an injection molding process.

§ Stamper

○ Method of production of hierarchical and convexo-concave-patterned stamper (stamper on which primary convexo-concave pattern and secondary convexo-concave pattern be formed)

- A master stamper was produced by a photolithographic technique, and a Cu master stamper with the primary convexo-concave pattern being impressed was produced by a Cu-electroforming process.
- Wet etching was conducted for the surface of the Cu master stamper to form a roughened surface, and then, a stamper with a secondary convexo-concave pattern being impressed by a Ni-electroforming process.

  - Primary convexo-concave pattern

    - $\varphi$s= 0.1 (s= 20 $\mu$m, d= 30 $\mu$m, and pitch= 200 $\mu$m)

  - Secondary convexo-concave pattern

    - Ra/RSm= 264 x 10$^{-3}$ (Ra= 933 $\mu$m, and RSm= 3.5 $\mu$m)

○ Method of production of secondary convexo-concave patterned stamper

- Wet etching was conducted for a flat surface of a Cu master stamper to form a roughened surface, and then, a stamper with a secondary convexo-concave pattern being impressed by a Ni-electroforming process.

  - Ra/RSm= 264 x 10$^{-3}$ (Ra= 933 $\mu$m, and RSm= 3.5 $\mu$m)

§ Transfer molding

- The stamper was heated to a temperature of 230°C by infra-red radiation heating with a halogen lamp, impressed to the front edge of the nozzle for one second, followed by cooling to obtain a front edge of the nozzle to which the convexo-concave pattern formed on the surface of the stamper was transfer-molded.

§ Carbon fluoride plasma treatment

- After the transfer molding, carbon fluoride plasma treatment was carried out under the following condition:

  - Apparatus

    - Discharge type: Low atmospheric pressure surface wave plasma

- Electric power source: 1500 W@2.45GHz

- Condition

  - Degree of vacuum: 4 Pa
  - Material gas: $CF_4$ 100 sccm
  - Duration of plasma treatment: 20 seconds

(2) Performance evaluation

§ Evaluation on convexo-concave shape

- Method of measurement
- As to the edge of the nozzle to which the convexo-concave pattern was transferred, measurement for the primary convexo-concave pattern and the secondary convexo-concave pattern were conducted by using a white-light interferometer, and measurement for the tertiary convexo-concave pattern was conducted by using an atomic force microscope (AFM). The area ratio $\varphi s$, arithmetic average roughness Ra, and average length RSm were calculated.
- Measurement condition of white-light interferometer
- Measurement device: New View 7300 manufactured by ZYGO Corporation
- Object lens magnification: 50-fold
- Ocular lens magnification: 2.0-fold
- Cutoff value of long wavelength side $\lambda c$=13.846155 $\mu$m
- Cutoff value of short wavelength side $\lambda s$=346.155 nm
- Measurement condition of AFM
- Measurement device: Nano Scope III manufactured by Veeco Instruments, Inc.
- Cutoff value of long wavelength side $\lambda c$=0.0824 $\mu$m

§ Determination of fluorine atom content

- Method of measurement
- Wide-band spectral analysis was conducted for the surface of the substrate by using an X-ray photoelectron spectrometer (XPS) to measure the amount of elements that exist on the surface, and the atomic ratio of fluorine atoms to carbon atoms (F/C) was calculated.
- Measurement device: K-Alpha manufactured by Thermo Fisher Scientific K.K.

§ Evaluations on small-quantity dripping quality and control performance of dripping quantity

- Testing method

  - The eye drop container main body was filled with real liquid, subjected to carbon fluoride plasma treatment, and capped with the nozzle formed by transfer molding.
  - Ten or more drops of the real liquid were dripped on a paper dish set on an electronic balance, and measured the total weight of the drops.
  - The weight of one drop was calculated by dividing the total amount of the drops by the number of the drops.

- Real liquid

  - C CUBE manufactured by RHOTO Pharmaceutical Co., Ltd.

- Measurement device: Even balance ML802 manufactured by Mettler-Toledo International Inc.
- Evaluation criterion

  - The case where the diameter of the opening and the dripping quantity are related with a positive correlation and the correlation coefficient r2 $\geqq$ 0.7, was judged as the nozzle have control performance of dripping quantity.
  - The case where the dripping quantity $\leqq$ 10 $\mu$L was judged as the nozzle have small-quantity dripping performance.

§ Evaluation on durability to repeated dripping

- Testing method

  - One hundred drippings were conducted to contaminate the surface.
  - The dripping quantity of the contaminated nozzle was measured.

- Real liquid

  - C CUBE manufactured by RHOTO Pharmaceutical Co., Ltd.

- Evaluation criterion

  - The case where the dripping quantity $\leqq$ 10 $\mu$L was judged as the nozzle have durability to repeated drippings.

[Second embodiment]

Next, examples of the nozzle according to the second embodiment of the present invention will be explained with reference to Table 2.

[Table 2]

| | | | Examples | | | | | | Com. Examples | |
|---|---|---|---|---|---|---|---|---|---|---|
| | | | (1) | (2) | (3) | (4) | (5) | (6) | (1) | (2) |
| | Drawings to be referred | | Fig. 5-a | Fig. 5-b | Fig. 5-d | Fig. 5-e | Fig. 5-f | Fig. 5-g | Fig. 3-a | Fig. 3-b |
| Molding method | Nozzle base | | 1 | 2 | 2 | 1 | 1 | 3 | 3 | 3 |
| | First dripping part | | a | b | b | c | d | - | - | - |
| | Protruding part fabricating step | | ○ | ○ | × | ○ | ○ | - | - | - |
| | Roughening step | | ○ | ○ | ○ | ○ | ○ | ○ | × | ○ |
| | Fluorinating plasma treatment step | | ○ | ○ | ○ | ○ | ○ | ○ | × | ○ |
| | Burrs-forming step | | × | × | × | × | × | ○ | × | × |
| Evaluation of performance | Small-quantity dripping performance | | ○ (5.0μL) | ○ (7.0μL) | ○ (4.0μL) | ○ (4.4μL) | ○ (9.2μL.) | ○ (4.9μL) | × (40μL) | ○ (3.7μL) |
| | Reproducibility of dripping quantity | | O (3.0%) | ○ (3.1%) | △ (4.2%) | ○ (3.0%) | ○ (3.1%) | ○ (3.2%) | × (18%) | × (6.2%) |
| | Overall evaluation | | ○ | ○ | △ | ○ | ○ | ○ | × | × |

(Nozzle base 1)

- Resin: Polyethylene (LJ8041 manufactured by JAPAN POLYETHYLENE CORPORATION)
- Molding method: injection molding method
- Outer shape: 6 mm in diameter x 5 mm in length
- Diameter of opening: 0.8 mm

(Nozzle base 2)

- Resin: Polyethylene (LJ8041 manufactured by JAPAN POLYETHYLENE CORPORATION)
- Molding method: injection molding method
- Outer shape: 6 mm in diameter x 5 mm in length
- Diameter of opening: 1.2 mm

(Nozzle base 3)

- Resin: Polyethylene (LJ8041 manufactured by JAPAN POLYETHYLENE CORPORATION)
- Molding method: injection molding method
- Outer shape: 6 mm in diameter x 5 mm in length
- Diameter of opening: 0.4 mm

(First dripping part a)

- Resin: Polyethylene (LJ8041 manufactured by JAPAN POLYETHYLENE CORPORATION)
- Molding method: injection molding method
- Outer shape: 0.8 mm in diameter x 0.4 mm in inner diameter x 1 mm in length
- Shape of first surface: rectangle

(First dripping part b)

- Resin: Polyethylene (LJ8041 manufactured by JAPAN POLYETHYLENE CORPORATION)
- Molding method: injection molding method
- Outer shape: 1.2 mm in diameter x 0.4 mm in inner diameter x 1 mm in length
- Shape of first surface: rectangle

(First dripping part c)

- Resin: Polyethylene (LJ8041 manufactured by JAPAN POLYETHYLENE CORPORATION)
- Molding method: injection molding method
- Outer shape: 0.8 mm in diameter x 0.4 mm in inner diameter x 1 mm in length
- Shape of first surface: reducing taper (tip angle: 30°)

(First dripping part d)

- Resin: Polyethylene (LJ8041 manufactured by JAPAN POLYETHYLENE CORPORATION)
- Molding method: injection molding method
- Outer shape: 0.8 mm in diameter x 0.4 mm in inner diameter x 1 mm in length
- Shape of first surface: increasing taper (tip angle: 45°)

(Surface-roughening step)

- A stamper made of Ni, on which a hierarchized convexo-concave structure was formed, was heated to a temperature of 230°C and hot-pressed to the nozzle base to form a roughened surface.

- Primary convexo-concave structure

- Line & space pattern
- Area ratio= 0.2

- Secondary convexo-concave structure

  - Ra/Rsm= $250 \times 10^{-3}$

(Fluorine plasma treatment step)

- Surface wave plasma device
- Power outlet: 1.5 kW@2.45GHz
- Material gas: $CF_4$ 200 sccm
- Degree of vacuum: 4 Pa
- Duration of treatment: 20 seconds

(Burrs-forming step)

- The opening of the nozzle base was knocked a hole by a drill having a diameter of 0.4 mm to form burrs.

(Protruding part fabrication step)

- The first dripping part was inserted into the opening of the nozzle base.
- In the fabrication, the dripping part was protruded from the surface of the nozzle base by 0.3 mm.

(Dripping test)

- The nozzle was inserted to a main body of an container for instillation of eye drops filled with ROHTO C CUBE.
- The container main body was pushed to drip 20 drops of the filling fluid, and the total amount of the 20 drops was measured.

(Evaluations on small-quantity dripping performance)

- An average amount value of the drops was calculated and the small quantity-dripping performance was evaluated.

  - ○ : Average amount value $\leqq$ 10 $\mu$L
  - × : Average amount value > 10 $\mu$L.

(Evaluation on reproducibility of dripping quantity)

- An average amount of drops and the standard deviation were calculated, and CV was calculated by means of the following expression to conduct the evaluation:

  - CV (%) = (Standard deviation)/(Average value) x 100
  - ○ : CV $\leqq$ 3.3 %
  - Δ : 3.3 < CV $\leqq$ 5.0 %
  - × : CV > 5.0 %

As above, the nozzle of the present invention is explained with reference to the preferred embodiments. However, the present invention is not restricted to the above-mentioned embodiments, and it is needless to say that various kinds of modifications within the scope of the present invention are applicable.

For instance, in the above-mentioned embodiments, a container for instillation of eye drops is described as an application object of the nozzle of the present invention. However, the application object is not limited to the container for instillation of eye drops. Namely, the nozzle of the present invention can also be used for a nozzle or inlet, other than the container for instillation of eye drops, that is desired to drip a fluid in a predetermined quantity per drop. For example, the nozzle of the present invention can be used for a container for a medicine other than eye drops, a container for seasoning such as soy source or source, a container for chemical product such as detergent or cosmetics, and the like; a nozzle for various kinds of containers, a nozzle for medical apparatus or laboratory instrument, and a nozzle of a device for dripping a liquid.

Industrial Applicability

The nozzle of the present invention can be suitably used as a nozzle for dripping fluid in a small amount, for example, for a container for instillation of eye drops or the like.

Description of referential numerals

| 1 | Container for instillation of eye drops |
|---|---|
| 2 | Container main body |
| 10, 10A, 10B | Nozzle |
| 11A | Front edge part |
| 11Aa | Opening |
| 11B | First dripping part |
| 11Ba | First surface |
| 12A | Side surface part |
| 12B | Second dripping part |
| 12Ba | Second surface |
| 14 | Bulwark |
| 15 | Thick wall part |
| 20 | Cap |
| 21 | Nozzle-abutting part |
| 100 | Roughened surface |
| 160 | Primary convexo-concave surface |
| 160a | Concave part |
| 160b | Convex part |
| 165 | Secondary convexo-concave surface |
| 170 | Liquid droplet |

**Claims**

1.  A nozzle composed of a non-fluorine-based resin,
    wherein fluorine atoms are incorporated into a molecular chain of the non-fluorine-based resin constituting a surface of the nozzle.

2.  The nozzle according to claim 1, wherein a front end part of the nozzle is roughened.

3.  The nozzle according to claim 2, wherein the roughened surface has a primary concavo-convex surface and a secondary concavo-convex surface formed in the primary concavo-convex surface and has finer concavities and convexities than those of the primary concavo-convex, and wherein the fluorine atoms are incorporated into the molecular chain of the non-fluorine-based resin.

4.  The nozzle according to claim 3, wherein a further fine tertiary concavo-convex surface is formed in the second concavo-convex surface, and the fluorine atom is incorporated into a molecular chain of the non-fluorine-based resin constituting the tertiary concavo-convex surface.

5.  The nozzle according to claim 2, wherein, when an area ratio (projection area of the solid-liquid interface per unit area an area ratio is taken as $\varphi_s$, the roughened surface satisfies $0.05 \leqq \varphi s \leqq 0.8$.

6.  The nozzle according to claim 5, wherein the roughened surface is formed of rectangular concavities and convexities.

7.  The nozzle according to claim 2, wherein, when an arithmetic average surface roughness representing an amplitude of the concavo-convex structure is taken as Ra and an average length corresponding to 1/2 pitch $R_0$ is taken as RSm, Ra and RSm satisfy $Ra/RSm \geqq 50 \times 10^{-3}$.

8.  The nozzle according to claim 1, wherein a circumferential protruded part is provided on an inner front end part of the nozzle.

9.  A nozzle wherein a surface of a front end part of the nozzle is provided with a first surface positioned nearer to the center of the nozzle and a second surface continuing to the outer peripheral side of the first surface, and the first surface and the second surface are composed of surfaces differing in surface free energy.

10. The nozzle according to claim 9, wherein the second surface has higher liquid repellency than that of the first surface.

11. The nozzle according to claim 9, wherein the second surface has different surface free energy from that of the first surface by being subjected to a surface roughening treatment.

12. The nozzle according to claim 9, wherein the nozzle is a nozzle for allowing liquid droplets to drip, and is provided with a first dripping part through which a dripped liquid is passed and a second dripping part arranged on the outer peripheral side of the first dripping part, and wherein
    a surface of the front end part of the first dripping part is the first surface and a surface of the front end part of the second dripping part is the second surface.

13. The nozzle according to claim 9, wherein the first surface protrudes in the pouring direction from the second surface.

14. The nozzle according to claim 9, wherein the first surface does not protrude in the pouring direction than the second surface.

15. The nozzle according to claim 9, wherein the shape of the first surface in a cross section including the central line of the nozzle is rectangular.

16. The nozzle according to claim 9, wherein the shape of the first surface in a cross section including the central line of the nozzle is a tapered shape that reduces in width in the pouring direction or a tapered shape that expands in width in the pouring direction.

17. The nozzle according to claim 1 or 9, which is provided with a cap that covers the nozzle without contacting the front end part of the nozzle.

18. The nozzle according to claim 17, wherein the inner surface of the cap covers the front end part of the nozzle by contacting a side surface continuing the front end part of the nozzle.

19. The nozzle according to claim 17, wherein the inner surface of the cap blocks the opening at the front end part by pressing the side surface continuing the front end part of the nozzle.

20. The nozzle according to claim 17, which comprises a cap that covers the nozzle without contacting the front end part of the nozzle, and the inner surface of the cap is provided with a cap that seals the nozzle by contacting the first surface.

21. The nozzle according to claim 1 or 9, wherein it comprises a thick wall part that outwardly protrudes from the outer peripheral edge of the front end part of the nozzle, and
    the thick wall part is formed by heat pressing of the front end part of the nozzle.

Fig.1

(a)

10(10A)

(b)

12A — — 12A

11Aa     11A

Fig.2

(a)

10(10B)

(b)

Fig.3

(a)

(b)

Fig.4

(a)

(b)

(c)

(d)

Fig.4

Fig.5

Fig.6

(a)

12B

11B

(b)

12B

Fig.7

−CF− or −CF₂−

100

Fluorine plasma

d

S

Fig.8

(a)

R

Air pocket

100

θ※

Cassie mode

Liquid droplets

(b)

R

θ※

Wenzel mode

Liquid droplets

Fig.9

Fig.10

Fluorine plasma

160
160a  160b

100

Fluorine plasma

170

d

S

Fig.11

(a)

20

10A

1

(b)

10A

20

21

Fig.12

(a)

10A

20

(b)

10A

20

1

1

(c)

10A

11A

20

21

Fig.13

(a)

20

10B

(b)

10B

12B

11B

20

21

Fig.14

(a)

1

10B

20

(b)

1

10B

20

(c)

10B

12B

11B

20

21

Fig.15

(a)

10B

20

22

1

(b)

10B

20

22

Fig.16

Fig.17

CF$_4$ or SiF$_4$

201

10

200

Fig.18

Fig.19

(1)　　　　　　　　　　(2)　　　　　(3)　　　　　(4)

12B

12B

12B

Fluorine plasma

Fig.20

(a)

(b)

Fig.21

(a)

(b)

(c)

Fig.22

(a)

(b)

(c)

Fig.23

Fig.24

$$\theta^* = \theta_E + (2\pi - \alpha)$$

15

L

$\theta_E$

$\alpha$

10

Fig.25

(a)

(b)

(c)

## INTERNATIONAL SEARCH REPORT

| International application No. |
| --- |
| PCT/JP2016/001135 |

A. CLASSIFICATION OF SUBJECT MATTER
*B65D47/18*(2006.01)i, *A61J1/05*(2006.01)i, *B65D47/06*(2006.01)i

According to International Patent Classification (IPC) or to both national classification and IPC

B. FIELDS SEARCHED

Minimum documentation searched (classification system followed by classification symbols)
B65D47/18, A61J1/05, B65D47/06

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 8-266587 A  (Taisho Pharmaceutical Co., Ltd.), 15 October 1996 (15.10.1996), entire text; all drawings (Family: none) | 1-21 |
| A | JP 2009-220843 A  (Tokuyama Dental Corp.), 01 October 2009 (01.10.2009), entire text; all drawings (Family: none) | 1-21 |

☐ Further documents are listed in the continuation of Box C.   ☐ See patent family annex.

| | |
| --- | --- |
| *      Special categories of cited documents: | "T"   later document published after the international filing date or priority date and not in conflict with the application but cited to understand the principle or theory underlying the invention |
| "A"   document defining the general state of the art which is not considered    to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 17 May 2016 (17.05.16) | 31 May 2016 (31.05.16) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014123140 A
- JP 2011105339 A